# EUROPEAN PATENT SPECIFICATION

(11) **EP 0 870 026 B1**
(45) Date of publication and mention of the grant of the patent: **19.05.2004**
(21) Application number: 96945295.2
(22) Date of filing: 19.12.1996
(51) Int. Cl.: C12N 15/16, C07K 14/575, A61K 38/22, C12N 15/70, C12N 1/21

(54) **OB PROTEIN DERIVATIVES HAVING PROLONGED HALF-LIFE**
OB PROTEINDERIVATE MIT VERLÄNGERTER HALBWERTZEIT
DERIVES DE PROTEINES OB A DEMI-VIE PROLONGEE

(30) Priority: 27.12.1995 US 579494; 20.06.1996 US 667184
(43) Date of publication of application: 14.10.1998
(73) Proprietor: Genentech, Inc., South San Francisco, CA 94080-4990 (US)
(72) Inventor: DE SAUVAGE, Frederic, J., Foster City, CA 94404 (US); LEVIN, Nancy, San Francisco, CA 94117 (US); VANDLEN, Richard, L., Hillsborough, CA 94010 (US)
(74) Representative: Kremer, Simon Mark
(86) International application number: PCT/US1996/020718
(87) International publication number: WO 1997/024440

(56) References cited:
- EP-A- 0 741 187
- WO-A-96/05309
- WO-A-97/00319
- NATURE, vol. 372, no. 6505, 1 December 1994, pages 425-432, XP000602062 YIYING ZHANG ET AL: "POSITIONAL CLONING OF THE MOUSE OBESE GENE AND ITS HUMAN HOMOLOGUE"

## Description

### Field of the Invention

The invention concerns long half-life derivatives of the OB protein. In particular, the invention concerns OB protein-immunoglobulin chimeras, and other long half-life derivatives of the OB protein, and compositions comprising and methods for administering them. The invention further relates to a method for treating obesity by administering a long half-life variant of the OB protein, such as, an OB protein-immunoglobulin chimera.

### Background of the Invention

Obesity is the most common nutritional disorder which, according to recent epidemiologic studies. affects about one third of all Americans 20 years of age or older. Kuczmarski *et al.,* J. Am. Med. Assoc. 272, 205-11 (1994). Obesity is responsible for a variety of serious health problems, including cardiovascular disorders, type II diabetes, insulin-resistance, hypertension, hypertriglyceridemia, dyslipoproteinemia and some forms of cancer. Pi-Sunyer, F.X., Anns. Int, Med. 119, 655-60 (1993); Colfitz, G.A., Am. J. Clin. Nutr. 55. 503S-507S(1992). A single-genemutation (the obesity or *"ob"* mutation) has been shown to result in obesity and type II diabetes in mice. Friedman, Genomics 11. 1054-1062 (1991). Zhang *et al.,* Nature 372, 425-431 (1994) have recently reported the cloning and sequencing of the mouse *ob* gene and its human homologue, and suggested that the *ob* gene product may function as part of a signalling pathway from adipose tissue that acts to regulate the size of the body fat depot. Parabiosis experiments performed more than 20 years ago predicted that the genetically obese mouse containing two mutant copies of the *ob* gene *(ob*/*ob* mouse) does not produce a satiety factor which regulates its food intake, while the diabetic *(db*/*db)* mouse produces but does not respond to a satiety factor. Coleman and Hummal, Am. J. Physiol, 217, 1298-1304 (1969); Coleman. Diabetol 9, 294-98 (1973). Recent reports by three independent research teams have demonstrated that daily injections of recombinant OB protein inhibit food intake and reduce body weight and fat in grossly obese *ob*/*ob* mice but not in *db*/*db* mice (Pelleymounter *et al.,* Science 269, 540-43 [1995]; Halaas *et al.,* Science 269, 543-46 [1995]; Campfield *et al.,* Science 269, 546-49[1995]), suggesting that the *ob* protein is such a satiety factor as proposed in early cross-circulationstudies. The results of these first studies leave many questions unanswered, and show a number of as yet unresolved discrepancies. For example, while modest effects of daily injections of the *ob* protein on food intake and body weight were reported in lean mice, there was a significant reduction in body fat as assessed by carcass composition in one (Halaas *et al., supra)* but not in another (Pelleymounter *et al.*, *supra)* of these reports, despite equivalent decreases in body weight. Furthermore, Pelleymounter *et al., supra* observed that, for reasons unknown, *ob*/*ob* mice treated with a 0.1 mg/kg/day dose of the OB protein actually increased their body weight by 17.13 %, while the weight reduction in the obese mice that received a I mg/kg/day dose of *ob* was rather moderate. The receptor or receptors of the *ob* protein are as of yet unidentified. While the existence of peripheral receptors cannot be ruled out at this time, the recent report that an increased expression of the *ob* gene in adipose tissue ofmice with hypothalamic lesions does not result in a lean phenotype suggests that the OB protein does not act directly on fat cells. Maffei *et al.,* Proc, Natl. Acad. Sci. 92, 6957-60 (1995). Researchers suggest that at least one OB receptor is localized in the brain. The identification and expression cloning of a leptin receptor (OB-R) was reported by Tartaglia *et al.,* Cell 83, 1263-71 (1995). Various isoforms of a leptin receptor are described by Cioffi *et al.,* Nature Medicine 2, 585-89 (1996). A human hematopoetin receptor, which might be a receptor of the OB protein, is described in PCT application Publication No. WO 96/08510, published 21 March 1996. A receptor of the OB protein is disclosed in Tartaglia *et al.,* Cell 83, 1263-71 (1995).

### Summary of the Invention

The present invention is based on the observation that the OB protein is significantly more effective at reducing body weight and adipose tissue weight when delivered as a continuous subcutaneous infusion than when the same dose is delivered as a daily subcutaneous injection. The invention is further based on the unexpected finding that a chimeric protein, in which the OB polypeptide is fused to an immunoglobulin constant domain, is strikingly more potent in reducing the body weight and adipose depots than native human OB, when both proteins are administered by subcutaneous injection once a day. The latter observation is particularly surprising since the OB protein-immunoglobulin chimera due to its large molecular weight, is not expected to be able to cross the blood-brain barrier, and reach the OB receptor which has been believed to be located in the brain.

The invention concerns long half-life derivatives of an OB protein capable of reducing body weightand/or food intake in an individual treated. The invention further concerns compositions containing such derivatives, and their administration for reducing body weight and/or food intake.

In respect of contracting states BE, CH, LI, DE, DK, ES, FR, GB, IT and NL, the invention is as set out in the claims for these states. In respect of the remaining states, the invention is as set out in the claims for these remaining states.

In one aspect, the invention concerns chimeric polypeptides comprising an OB protein amino acid sequence capable of binding to a native OB receptor linked to an immunoglobulin sequence (briefly referred to as OB-immunoglobulinchimeras or immunoadhesins). In respect of contrating states BE, CH, LI, DE, DK, ES, FR, GB, IT and NL the chimeric polypeptide is modified with a hydrophilic non-proteinaceous polymer. In a specific embodiment, the chimeric polypeptides comprise a fusion of an OB amino acid sequence capable of binding a native OB receptor,to an immunoglobulin constant domain sequence. The OB portion of the chimeras of the present invention preferably has sufficient amino acid sequences from a native OB protein to retain the ability to bind to and signal through a native OB receptor. Most preferably, the OB protein retains the ability to reduce body weight when administered to obese human or non-human subjects. The OB polypeptide is preferably human, and the fusion is preferably with an immunoglobulin heavy chain constant domain sequence. In another aspect, the association of two OB polypeptide-immunoglobulin heavy chain fusions (e.g., via covalent linkage by disulfide bond(s)) results in a homodimeric immunoglobulin-like structure. An immunoglobulin light chain may further be associated with one or both of the OB-immunoglobulin chimeras in the disulfide-bonded dimer to yield a homotrimeric or homotetrameric structure.

The invention further concerns nucleic acid encoding chimeric polypeptide chains of the present invention, expression vectors containing DNA encoding such molecules, transformed host cells, and methods for the production of the molecules by cultivating transformant host cells.

Although the long half-life derivatives of the present invention are particularly useful for reducing body weight and/or food intake, they can generally be used for the treatment of conditions associated with the abnormal expression or function of the OB gene and/or to elicit biological responses mediated by an OB receptor. Thus, the OB derivatives of the present invention may be used to treat bulemia, to reduce insulin levels, e.g. in Type I or II diabetic patients, and as mitogens of various cell types expressing an OB receptor. All these and related uses are within the scope of the present invention.

In another aspect, the invention concerns a method for increasing the potency of an OB protein according to claim 19 in respect of BE, CH, LI, DE, DK, ES, FR, GB, IT and NL and claim 22 in respect of the remaining states.

### Brief Description of the Figures

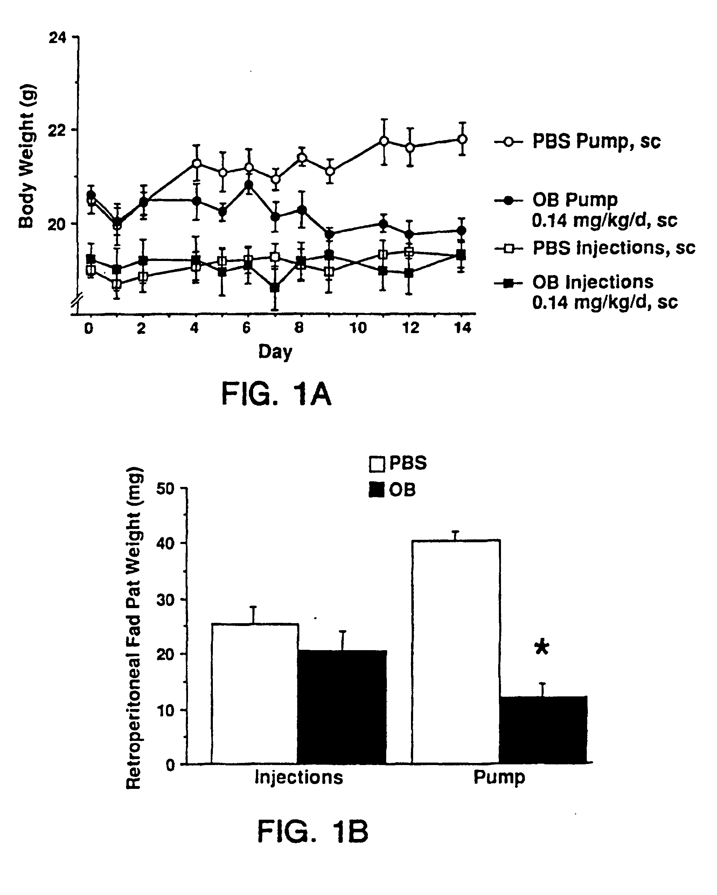

**Figure 1** top -- Lean female mice were treated with murine OB protein either as a continuous subcutaneo us infusion or daily subcutaneous injections. The data shown are the mean body weight of each group, in grams, n = 4 mice/point.
**Figure 1** bottom - The mean weight of the retroperitoneal fat pads are shown. Continuous subcutaneous infusions of the OB protein were also more effective than daily subcutaneous injections at reducing adipose tissue weight.

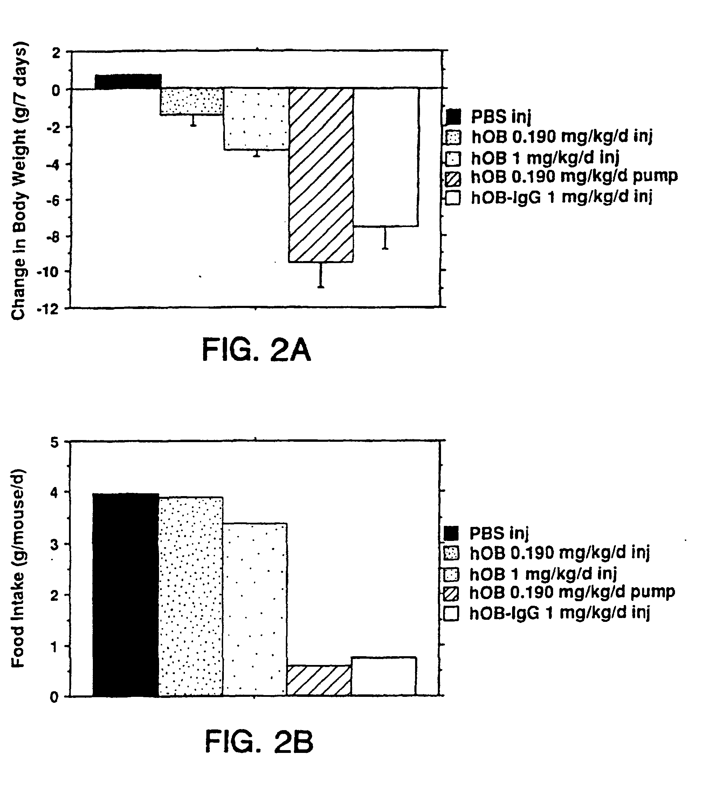

**Figure 2** top -- Obese female *ob*/*ob* mice were treated with human OB protein (hOB) or with a human OB-IgG-1 fusion protein (hOB-IgG-1). The data shown are the mean change in body weight for each treatment group from the first to the last day of experiment, in grams, n = 3 mice/bar except for the hOB 0.19 mg/kg/day by injection group, where n = 4, and PBS injection group, where n = 1.
**Figure 2** bottom -- The data shown were the mean food intake for each treatment group for the six 24 hour periods of the experiment, in grams/mouse/day, n = 1/bar.
**Figure 3** top and bottom -- Obese (*ob*/*ob*) female mice were treated with either hOB or the hOB-IgG-1 fusion protein by daily subcutaneous injections for 7 days. The data are depicted as in Figure 2, with n **=** 4 for all treatment groups.
**Figure 4** top - - Obese female *ob*/*ob* mice were treated with human protein (hOB) or with PEG-hOB. The data shown are the mean change in body weight for each treatment group from the first to the last day of experiment, in grams, n = 3-4 mice/bar except for the PBS injection group, where n = 1. The materials were injected daily subcutaneously. The "PEG IX" and "PEG 2X" refer to the ratio of the PEG reagent to protein in the preparation of the molecule.

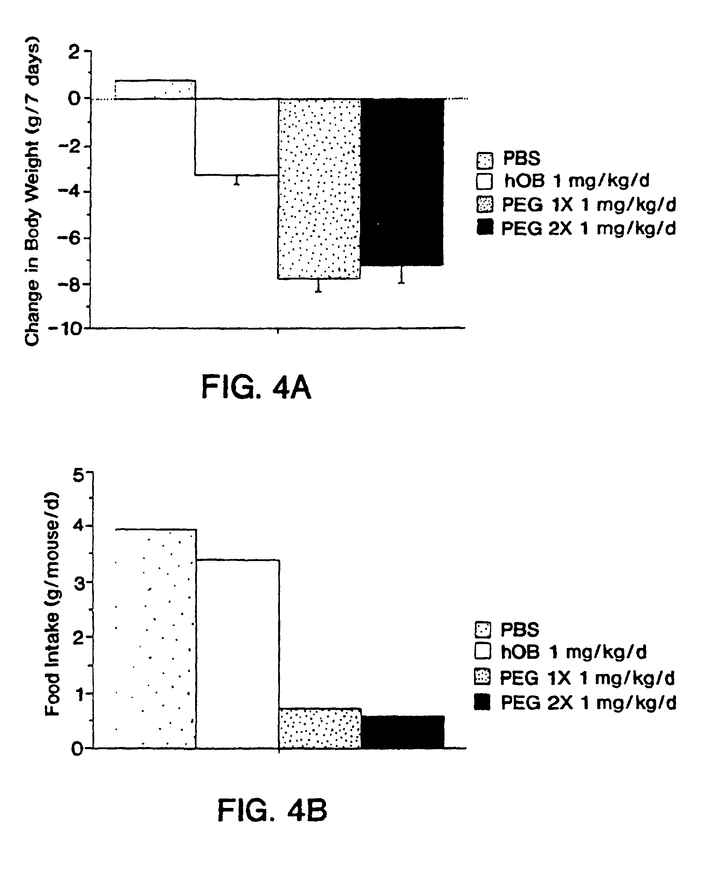

**Figure 4** bottom - - The data shown were the mean food intake for each treatment group for the six 24 hour periods of the experiment, in grams/mouse/day, n = 3-4/bar.

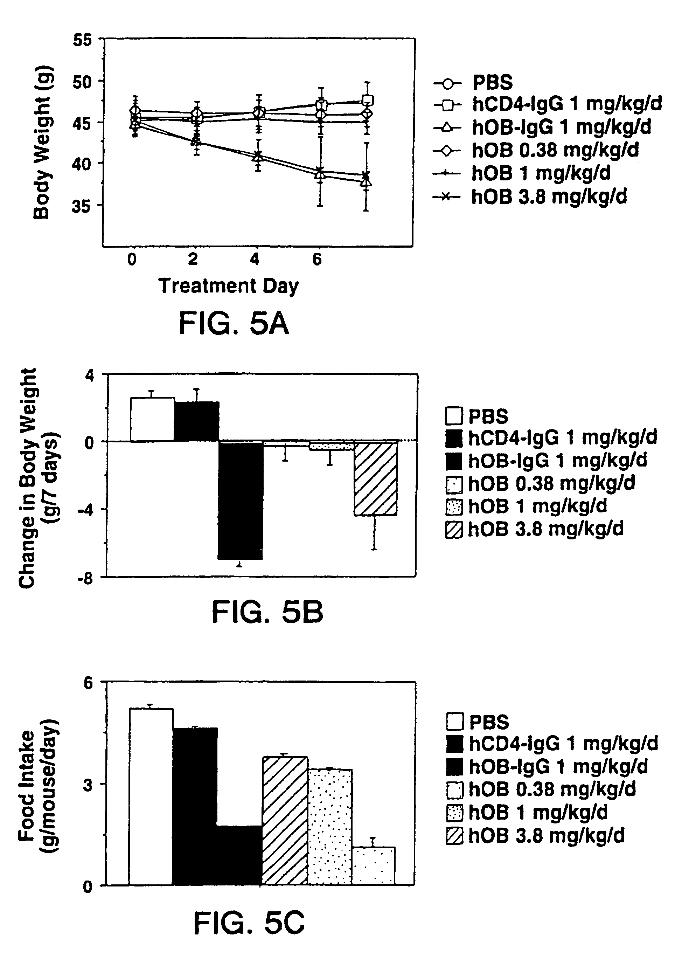

**Figure 5** - - Obese *(ob*/*ob)* female mice were treated with either the hOB-IgG fusion protein, native hOB, or hCD4-IgG by daily subcutaneous injections for 7 days. n = 6 for all treatment groups, except hOB at 3.8 mg/kg/d, where n = 2. Again it was observed that the fusion protein was more effective than the native hOB protein at reducing body weight (top and middle panels) and food intake (bottom panel).

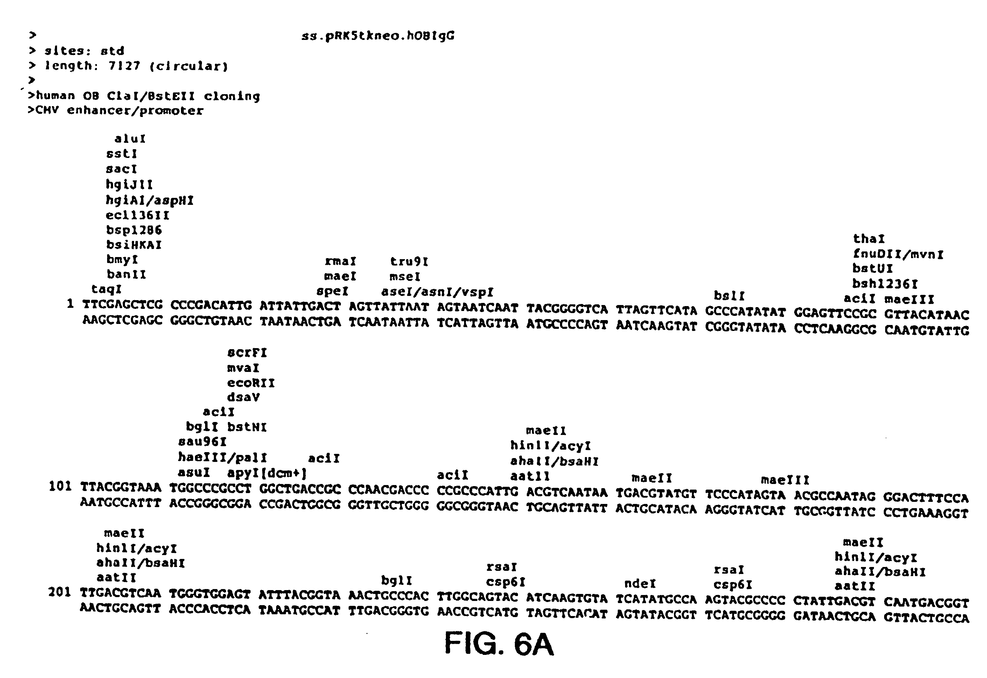

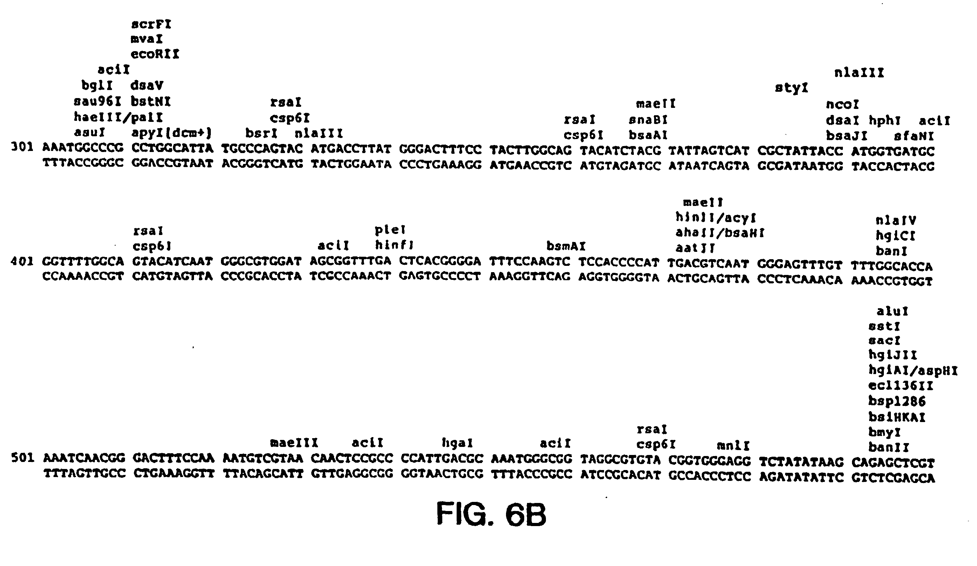

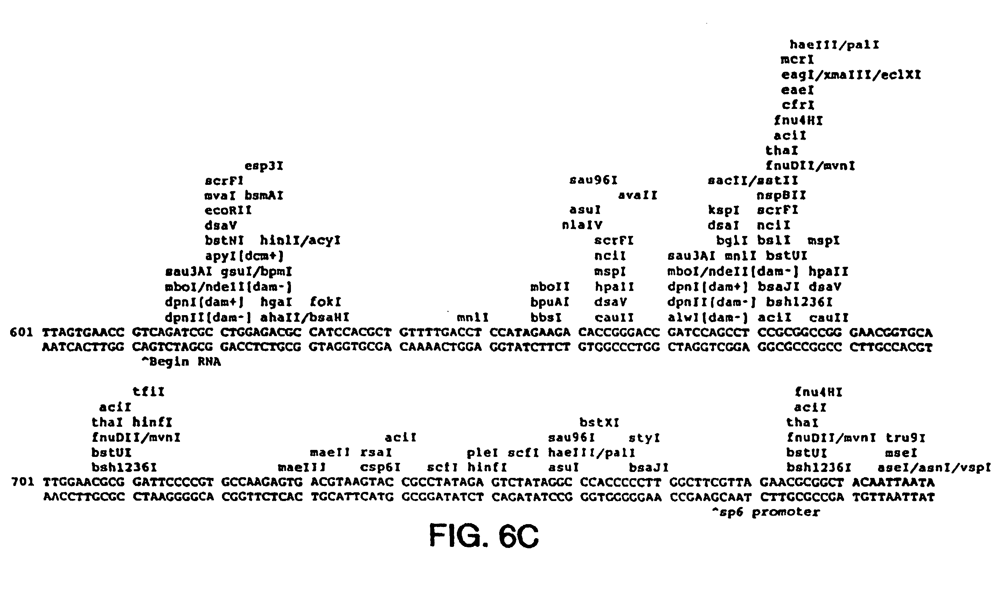

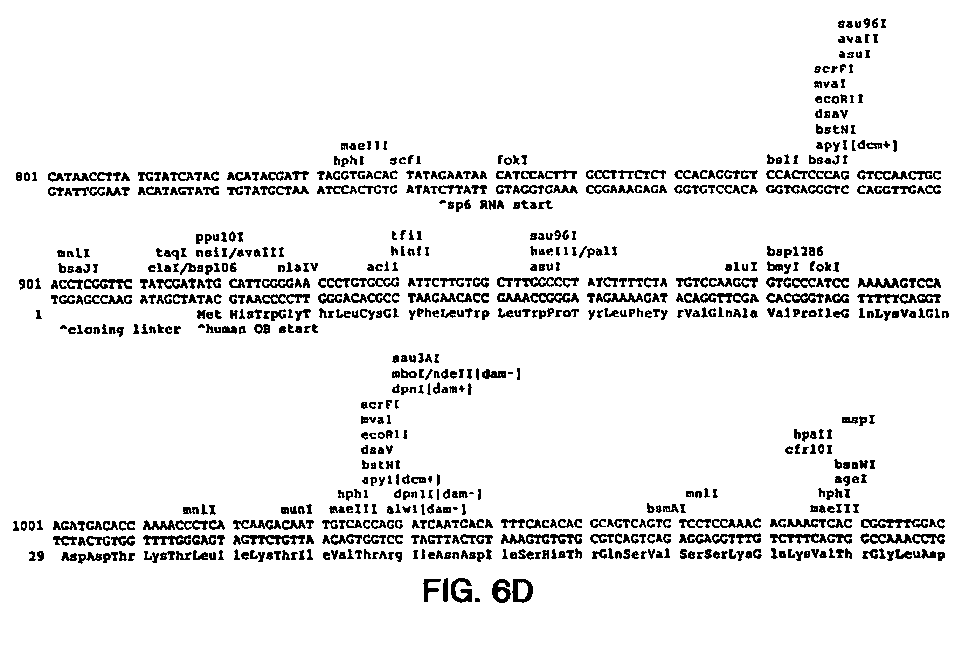

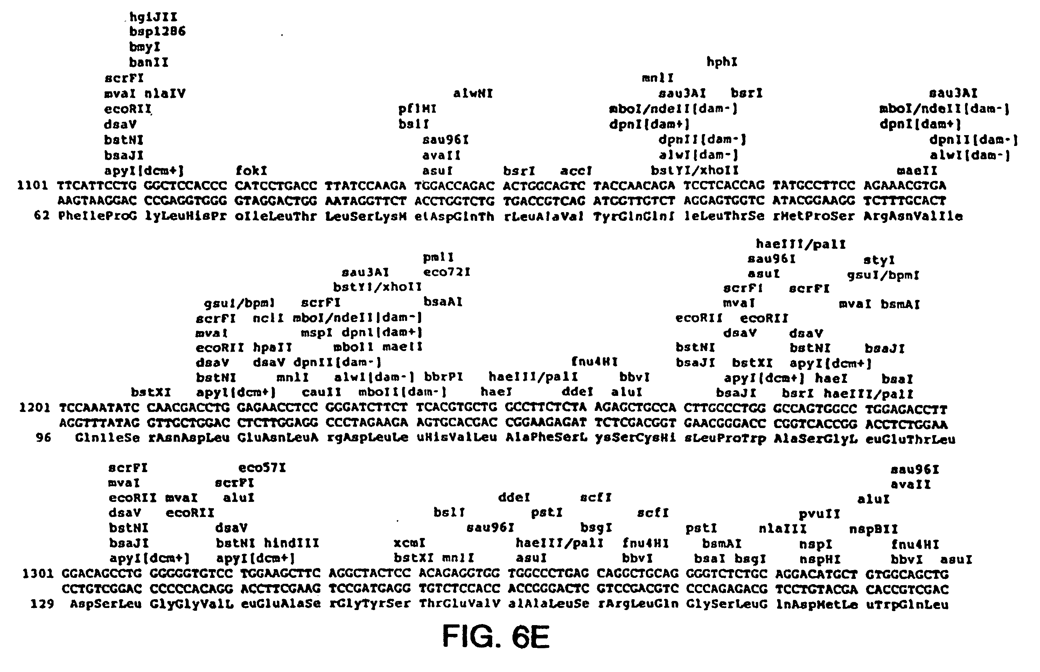

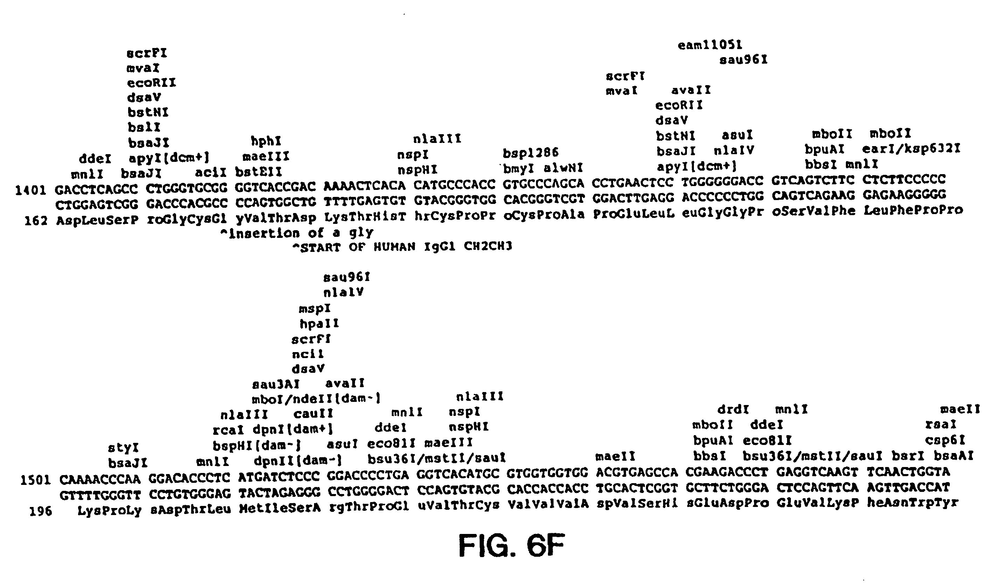

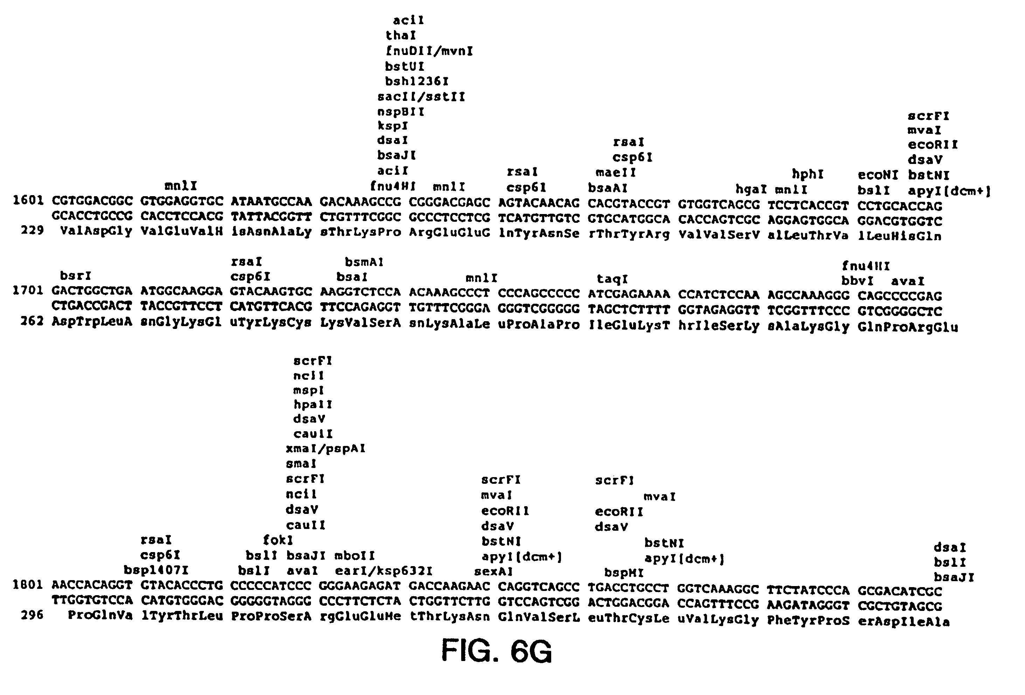

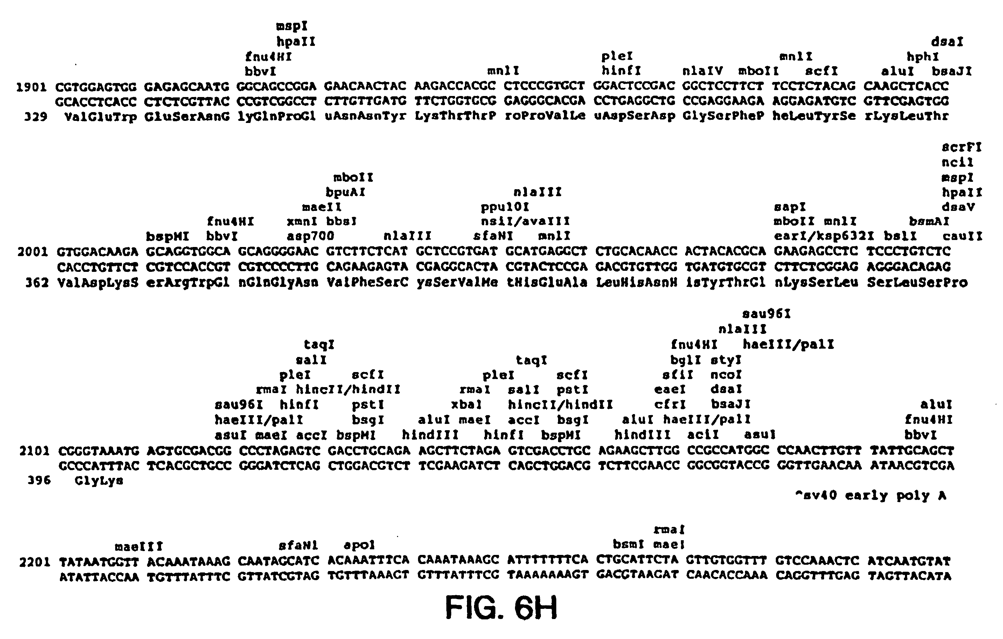

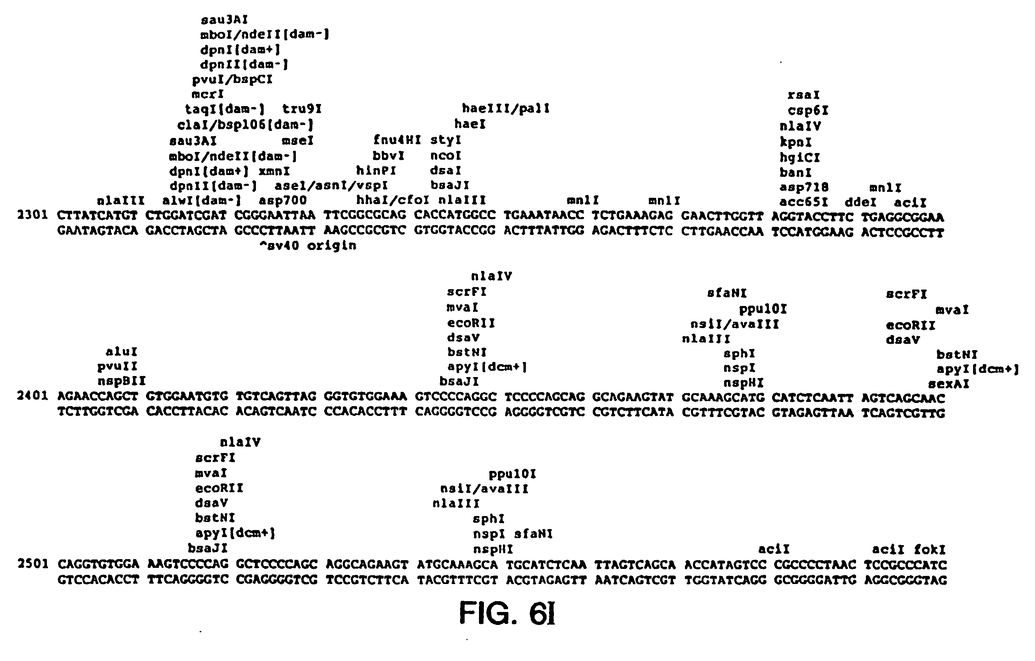

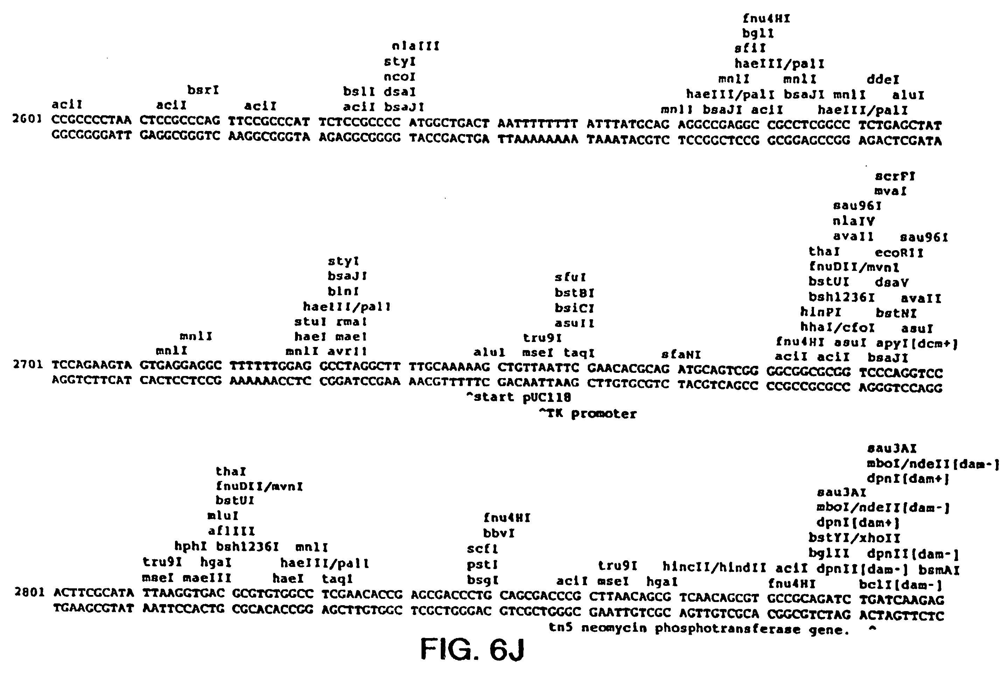

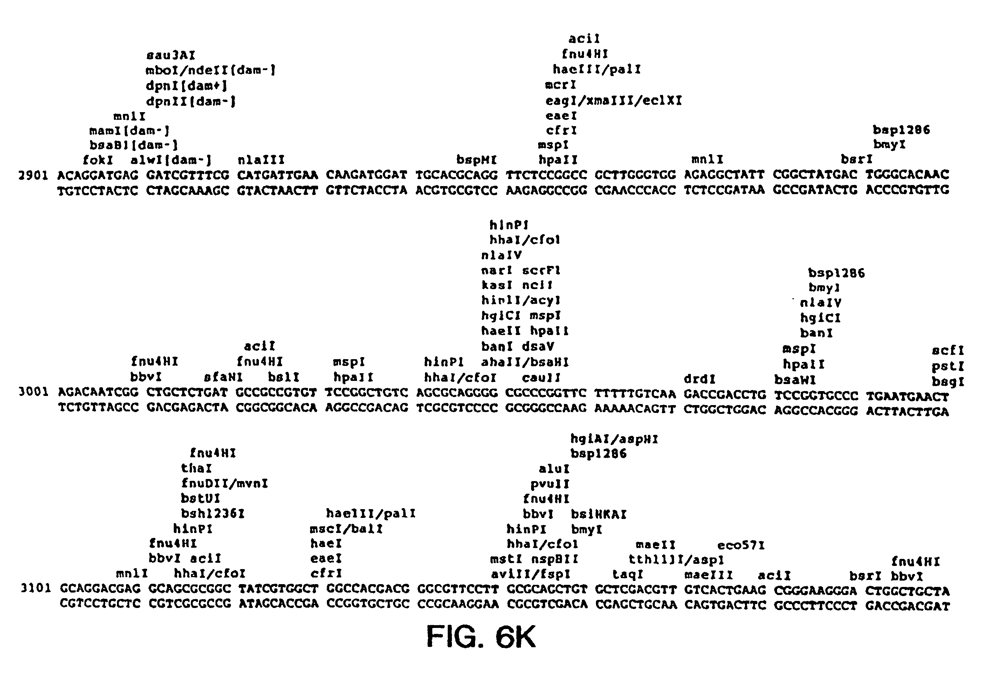

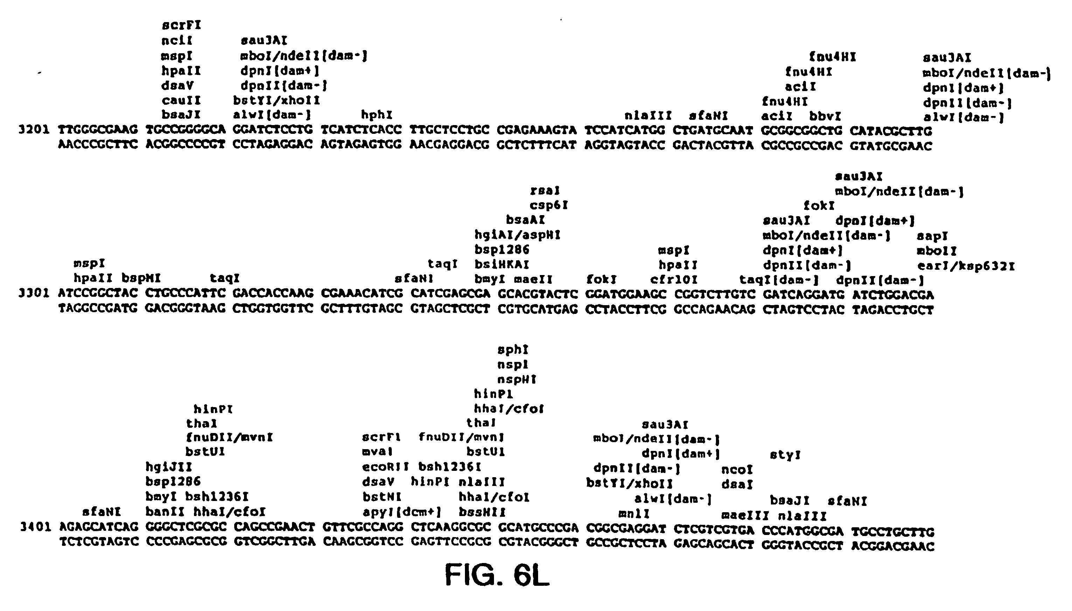

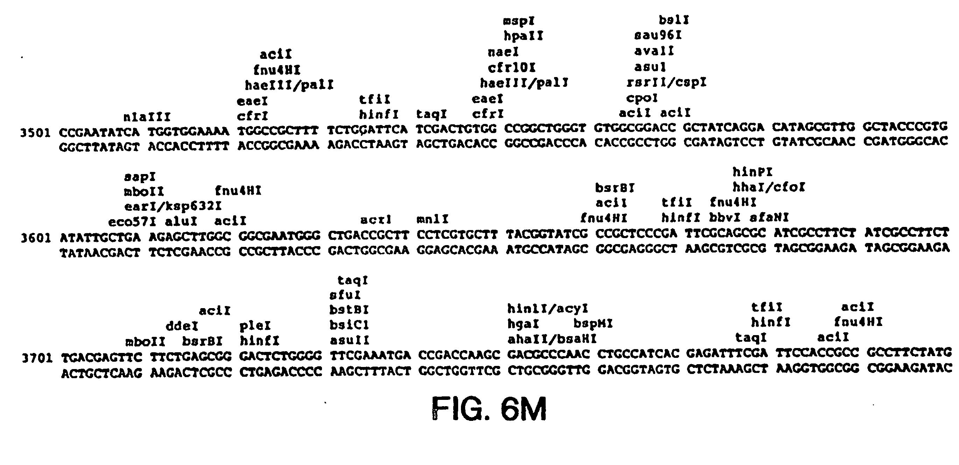

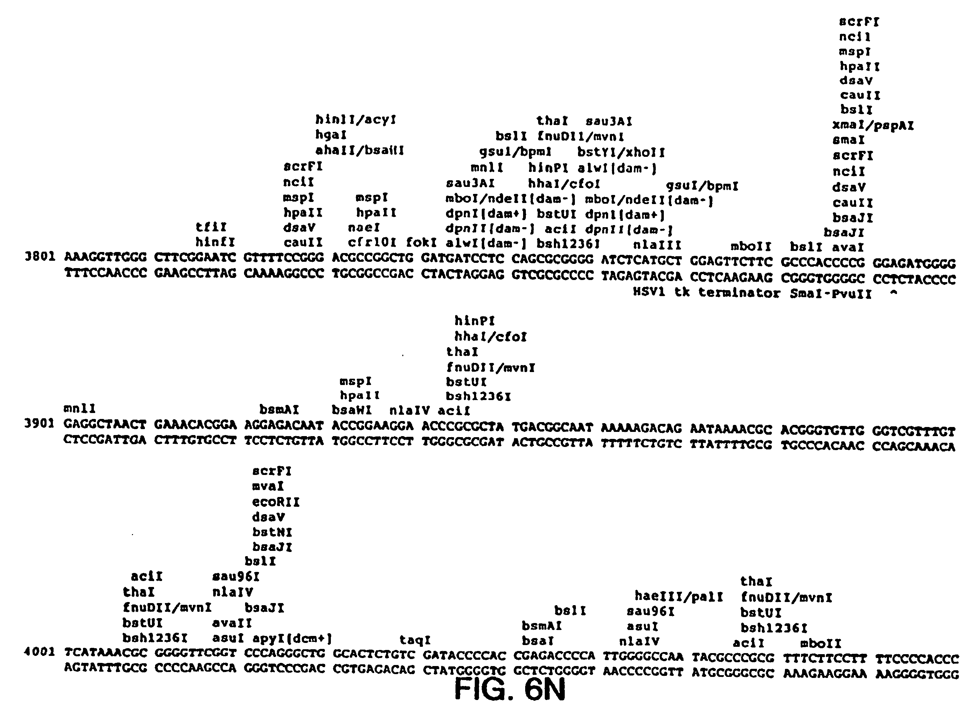

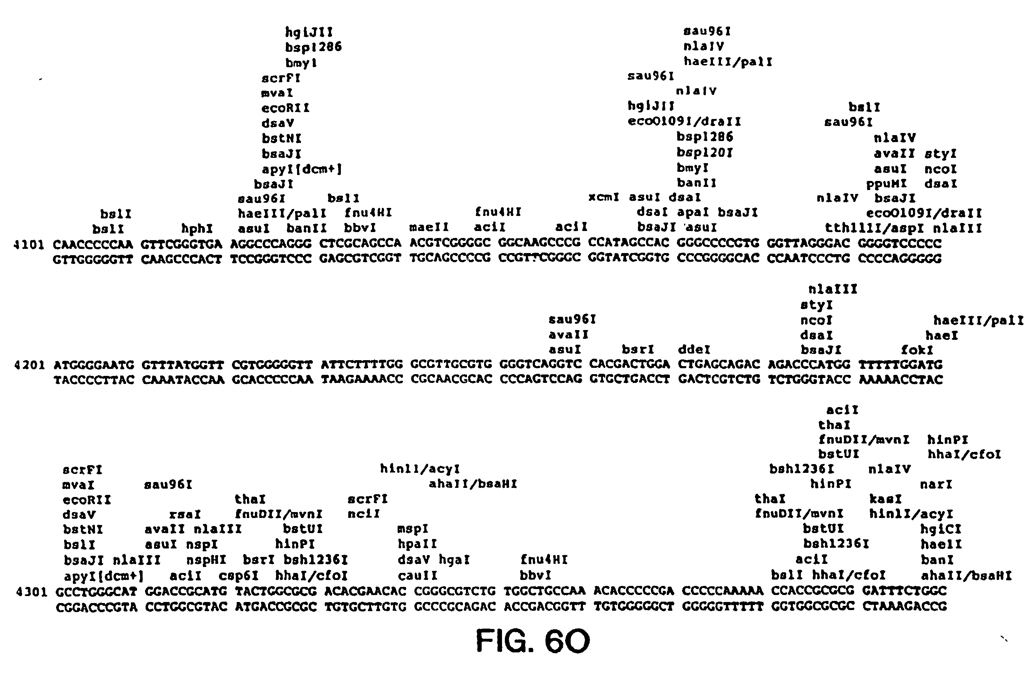

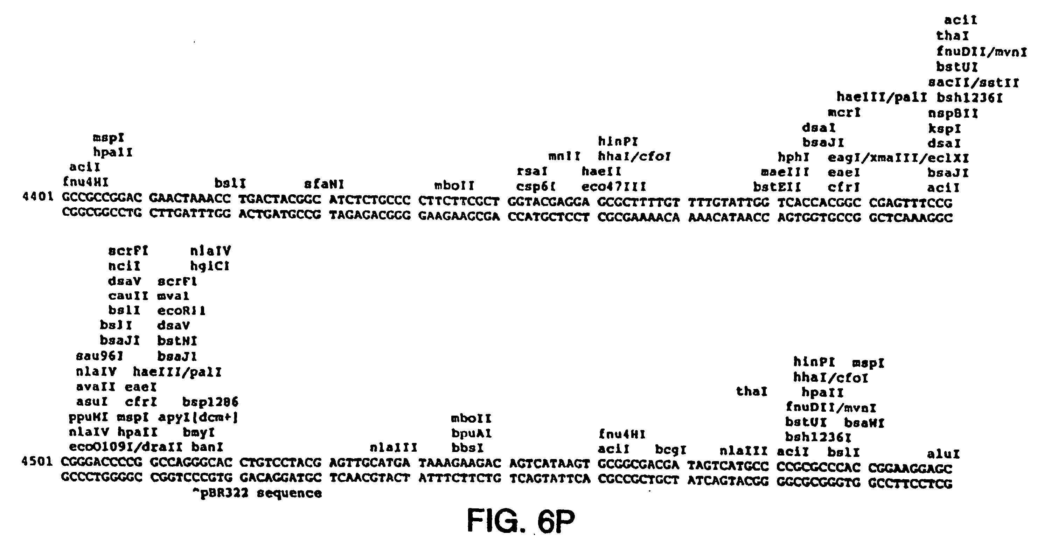

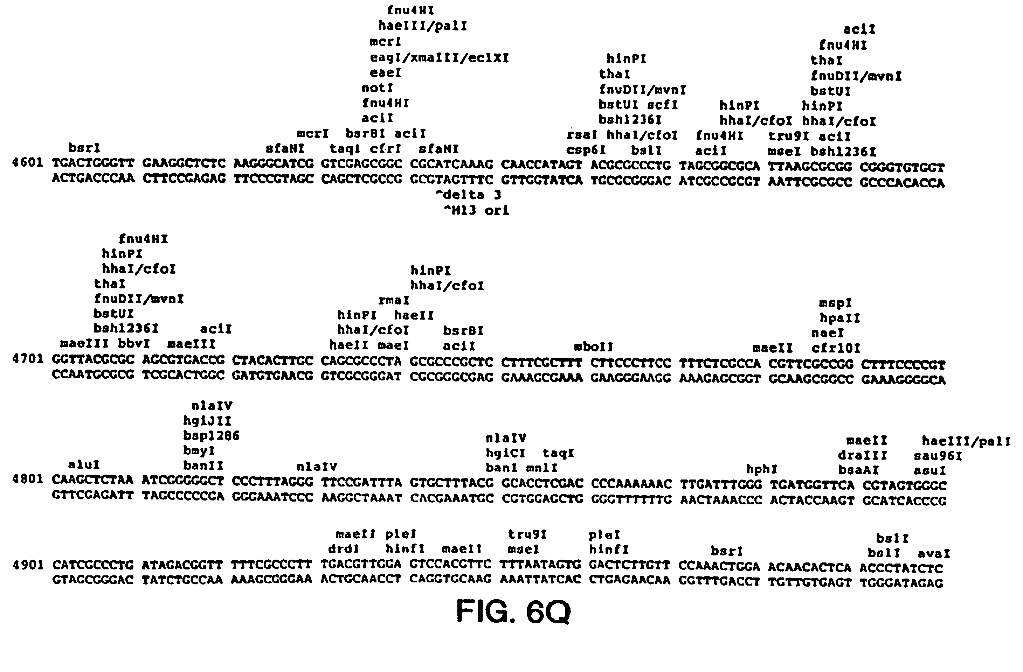

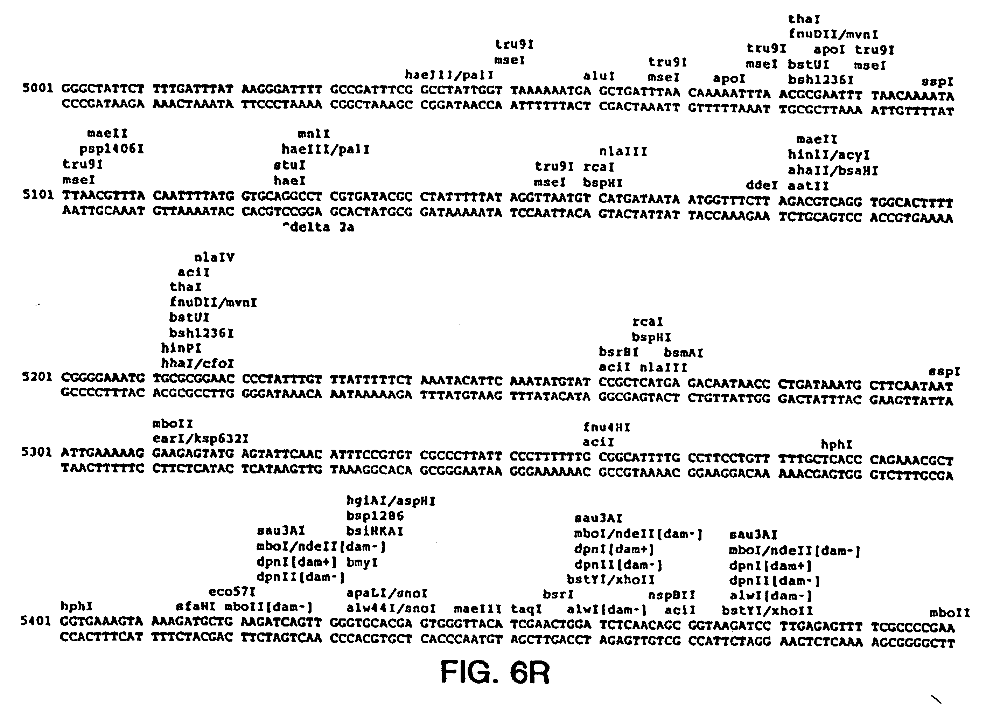

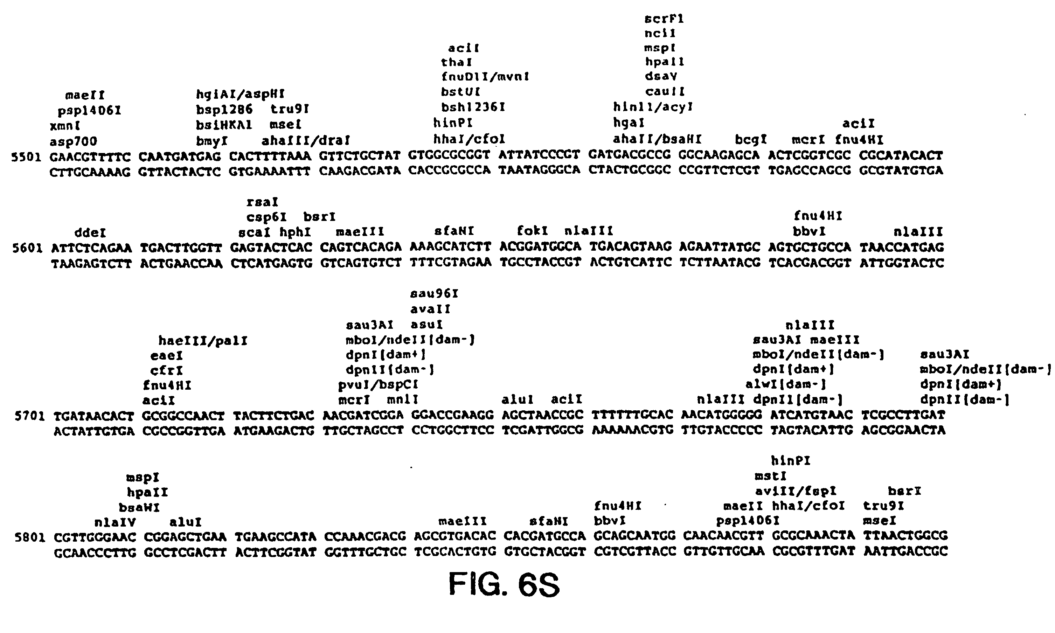

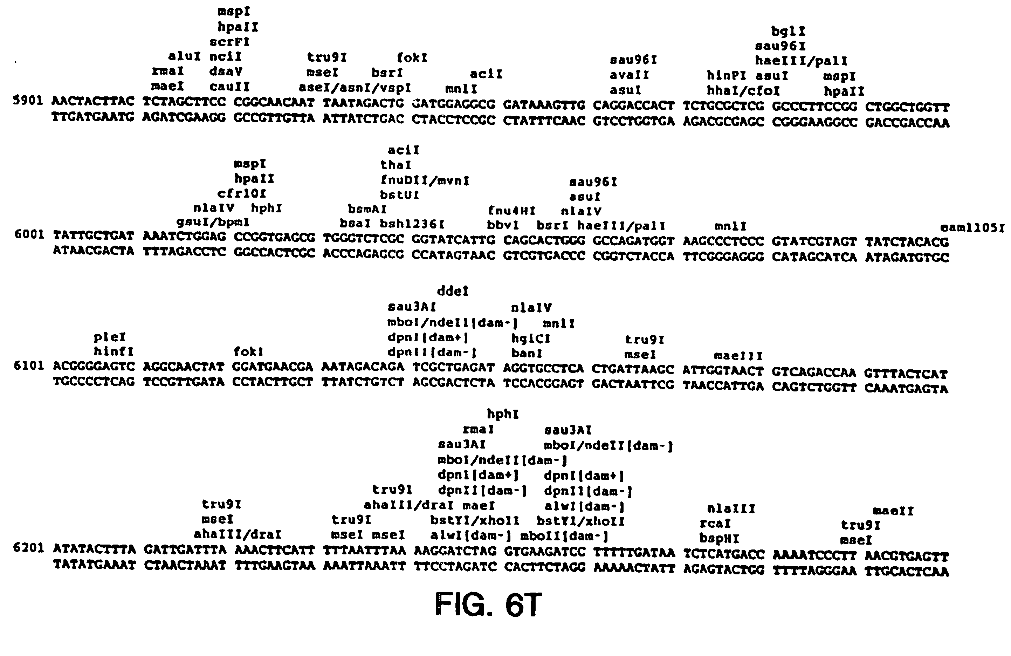

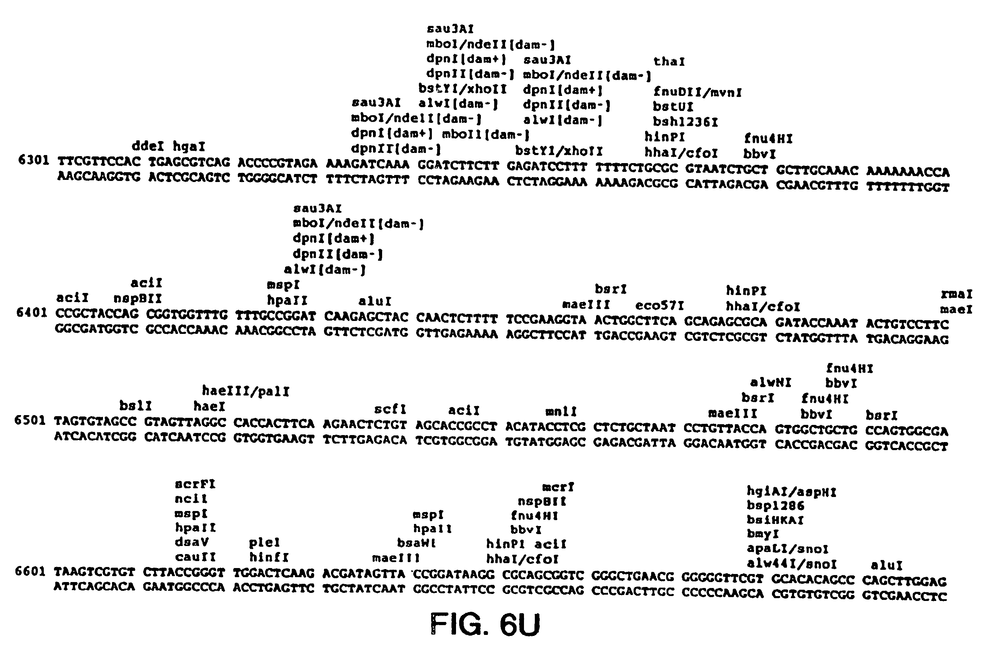

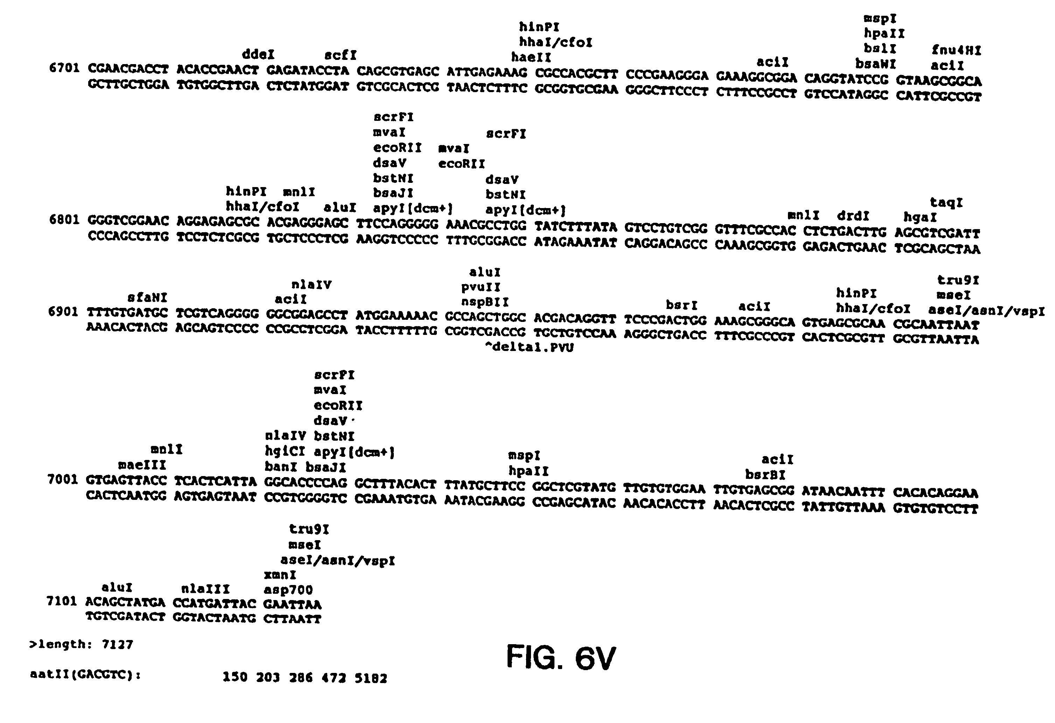

**Figure 6** - - The nucleotide sequence (SEQ. ID. NO:1) and the amino acid sequence (SEQ. ID. NO: 2) of the human OB-IgG-1 chimera of Example 1.

### Detailed Description of the invention

### A. Definitions

The term "obesity" is used to designate a condition of being overweight associated with excessive bodily fat. The desirable weight for a certain individual depends on a number of factors including sex, height, age, overall built, etc. The same factors will determine when an individual is considered obese. The determination of an optimum body weight for a given individual is well within the skill of an ordinary physician.

The phrase "long half-life" and grammatical variants thereof, as used in connection with OB derivatives, concerns OB derivatives having a longer plasma half-life and/or slower clearance than a corresponding native OB protein. The long half-life derivatives preferably will have a half-life at least about 1.5-times longer than a native OB protein; more preferably at least about 2-times longer than a native OB protein, more preferably at least about 3-time longer than a native OB protein. The native OB protein preferably is that of the individual to be treated.

The terms "OB", "OB polypeptide" , "OB protein" and their grammatical variants are used interchangeably and refer to "native" or "native sequence" OB proteins (also known as "leptins") and their functional derivatives. The OB polypeptides have the typical structural features of cytokines, i.e. polypeptides released by one cell population which act on another cell as intercellularmediators, such as, for example, growth hormones, insulin-like growth factors, interleukins, insulin, glycoprotein hormones such as, follicle stimulating hormone (FSH), thyroid stimulating hormone (TSH), tumor necrosis factor-α and -β (TNF-α and -β), nerve growth factors, such as NGF-β, PDGF, transforming growth factors (TGFs) such as, TGF-α and TGF-β, insulin-like growth factor-1 and -2 (IGF-1 and IGF-2), erythropoietin, osteoinductive factors, interferons (lFNs) such as, IFN-α, IFN-β and IFN-γ, colony stimulating factors (CSFs) such as, M-CSF, GM-CSF, and G-CSF, interleukins (ILs) such as, IL-1, IL-2, IL-3, IL-4, IL-5, IL-6, IL-7, IL-8 and other polypeptide factors.

The terms "native" and "native sequence" OB polypeptide are used to refer to an OB polypeptide from any animal species (e.g. human, murine. rabbit, cat, cow, sheep, chicken, porcine, equine, etc.), as occurring in nature, including naturally-occurringalleles, deletion, substitution and/or insertion variants, as currently known or as might be identified in the future, provided that they retain the ability to bind to and, preferably, signal through the OB receptor. Thus, a native human OB polypeptide includes the amino acid sequence between the N-terminus and the cysteine (Cys) at position 167 of the amino acid sequence shown in Figure 6 (see also SEQ. ID. NO: 2 and Figure 6 of Zhang *et al., supra),* and naturally occurring variants of this protein, as currently known or might be identified in the future. Similarly, a "native" or "native sequence" murine OB polypeptide has the amino acid sequence shown in Figure 6 of Zhang *et al., supra,* and naturally occurring variants of that polypeptide, as currently known or might be identified in the future. The definition specifically includes variants with or without a glutamine at amino acid position 49, using the amino acid numbering of Zhang *et al., supra.* The terms "native" and "native sequence" OB polypeptide include the native proteins with or without the initiating N-terminal methionine (Met), and with or without the native signal sequence, either in monomeric or in dimeric form. The native human and murine OB polypeptides known in the art are 167 amino acids long, contain two conserved cysteines, and have the features of a secreted protein. The polypeptide is largely hydrophilic, and the predicted signal sequence cleavage site is at position 21, using the amino acid numbering of Zhang *et al., supra.* The overall sequence homology of the human and murine sequences is about 84%. The two proteins show a more extensive identity in the N-terminal region of the mature protein, with only four conservative and three non-conservative substitutions among the residues between the signal sequence cleavage site and the conserved Cys at position 117. The molecular weight of OB proteins is about 16 kD in a monomeric form.

A "functional derivative" of a native polypeptide is a compound having a qualitative biological property in common with the native polypeptide. A functional derivative of an OB polypeptide is a compound that has a qualitative biological property in common with a native (human or non-human) OB polypeptide. "Functional derivatives" include, but are not limited to, fragments of native polypeptides from any animal species (including humans), and derivatives of native (human and non-human)polypeptidesand their fragments, provided that they have a biological activity in common with a corresponding native polypeptide.

"Fragments" comprise regions within the sequence of a mature native OB polypeptide. Preferred fragments of OB polypeptides include the C-terminus of the mature protein, and may contain relatively short deletion(s) at the N-tenninus and in other parts of the molecule not required for receptor binding and/or for structural integrity.

The term "derivative" is used to define amino acid sequence variants, and covalent modifications of a native polypeptide, whereas the term "variant" refers to amino acid sequence variants within this definition.

"Biological property" in the context of the definition of "functional derivatives" is defined as either 1) immunological cross-reactivity with at least one epitope of a native polypeptide (e.g. a native OB polypeptide of any species), or 2) the possession of at least one adhesive, regulatory or effector function qualitatively in common with a native polypeptide.

Preferably, the functional derivatives are polypeptides which have at least about 65% amino acid sequence identity, more preferably about 75% amino acid sequence identity, even more preferably at least about 85% amino acid sequence identity, most preferably at least about 95% amino acid sequence identity with a native polypeptide. In the context of the present invention, functional derivatives of native sequence human OB polypeptides preferably show at least 95% amino acid sequence identity with the native OB proteins, and are not immunogenic in the human.

Amino acid sequence identity or homology is defined herein as the percentage of amino acid residues in the candidate sequence that are identical with the residues of a corresponding native polypeptide sequence, after aligning the sequences and introducing gaps, if necessary, to achieve the maximum percent homology, and not considering any conservative substitutions as part of the sequence identity. Neither N- or C-tetminal extensions nor insertions shall be construed as reducing identity or homology.

Immunologically cross-reactive as used herein means that the candidate (poly)peptide is capable of competitively inhibiting the qualitative biological activity of a corresponding native polypeptide having this activity with polyclonal antibodies or antisera raised against the known active molecule. Such antibodies and antisera are prepared in conventional fashion by injecting an animal such as a goat or rabbit, for example, subcutaneously with the known native OB protein in complete Freud's adjuvant, followed by booster intraperitoneal or subcutaneous injection in incomplete Freud's.

The term "isoiated OB polypeptide" and grammatical variants thereof refer to OB polypeptides (as hereinabove defined) separated from contaminant polypeptides present in the human, other animal species, or in other source from which the polypeptide is isolated.

In general, the term "amino acid sequence variant" refers to molecules with some differences in their amino acid sequences as compared to a reference (e.g. native sequence) polypeptide. The amino acid alterations may be substitutions, insertions, deletions or any desired combinations of such changes in a native amino acid sequence.

Substitutional variants are those that have at least one amino acid residue in a native sequence removed and a differentamino acid inserted in its place at the same position. The substitutionsmay be single, where only one amino acid in the molecule has been substituted, or they may be multiple, where two or more amino acids have been substituted in the same molecule.

insertional variants are those with one or more amino acids inserted immediately adjacent to an amino acid at a particular position in a native amino acid sequence. Immediately adjacent to an amino acid means connected to either the α-carboxy or α-amino functional group of the amino acid.

Deletional variants are those with one or more amino acids in the native amino acid sequence removed. Ordinarily, deletional variants will have one or two amino acids deleted in a particular region of the molecule.

"Covalent derivatives" include modifications of a native polypeptide or a fragment thereof with an organic proteinaceousor non-proteinaceousderivatizing agent, and post-translational modifications. Covalent modifications are traditionally introduced by reacting targeted amino acid residues with an organic derivatizing agent that is capable of reacting with selected sites or terminal residues, or by harnessing mechanisms of post-translational modifications that function in selected recombinant host cells. Certain post-translational modifications are the result of the action of recombinant host cells on the expressed polypeptide. Glutaminyl and asparaginyl residues are frequently post-translationally deamidated to the corresponding glutamyl and aspartyl residues. Alternatively, these residues are deamidated under mildly acidic conditions. Either form of these residues may be present in the OB-immunoglobulin chimeras of the present invention. Other post-translational modifications include hydroxytation of proline and lysine, phosphorylation of hydroxyl groups of seryl, tyrosine or threonyl residues, methylation of the α-amino groups of lysine, arginine, and histidine side chains [T.E. Creighton, Proteins: Structure and Molecular Properties, W.H. Freeman & Co., San Francisco, pp. 79-86 (1983)].

The terms "DNA sequence encoding", "DNA encoding" and "nucleic acid encoding" refer to the order or sequence of deoxyribonucleotides along a strand of deoxyribonucleic acid. The order of these deoxyribonucleotides determinesthe order of amino acids along the polypeptide chain. The DNA sequence thus codes for the amino acid sequence.

The terms "replicable expression vector" and "expression vector" refer to a piece of DNA, usually double-stranded, which may have inserted into it a piece of foreign DNA. Foreign DNA is defined as heterologous DNA, which is DNA not naturally found in the host cell. The vector is used to transport the foreign or heterologous DNA into a suitable host cell. Once in the host cell, the vector can replicate independently of the host chromosomal DNA, and several copies of the vector and its inserted (foreign) DNA may be generated. In addition, the vector contains the necessary elements that permit translating the foreign DNA into a polypeptide. Many molecules of the polypeptide encoded by the foreign DNA can thus be rapidly synthesized.

The term "control sequences" refers to DNA sequences necessary for the expression of an operably linked coding sequence in a particular host organism. The control sequences that are suitable for prokaryotes, for example, include a promoter, optionally an operator sequence, a ribosome binding site, and possibly, other as yet poorly understood sequences. Eukaryotic cells are known to utilize promoters, polyadenylation signals, and enhancer.

Nucleic acid is "operably linked" when it is placed into a functional relationship with another nucleic acid sequence. For example, DNA for a presequence or a secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous and, in the case of a secretory leader, contiguous and in reading phase. However, enhancers do not have to be contiguous. Linking is accomplished by ligation at convenient restriction sites. If such sites do not exist, then synthetic oligonucleotide adaptors or linkers are used in accord with conventional practice.

In the context of the present invention the expressions "cell", "cell line", and "cell culture" are used interchangeably,and all such designations include progeny. Thus, the words "transformants" and "transformed (host) cells" include the primary subject cell and cultures derived therefrom without regard for the number of transfers. It is also understood that all progeny may not be precisely identical in DNA content, due to deliberate or inadvertent mutations. Mutant progeny that have the same function or biological activity as screened for in the originally transformed cell are included. Where distinct designations are intended, it will be clear from the context.

Native immunoglobulins are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies between the heavy chains of different immunoglobulin isotypes. Each heavy and light chain also has regularly spaced intrachain disulfide bridges. Each heavy chain has at one end a variable domain (V_{H}) followed by a number of constant domains. Each light chain has a variable domain at one and (V_{L}) and a constant domain at its other end; the constant domain of the light chain is aligned with the first constant domain of the heavy chain. and the light chain variable domain is aligned with the variable domain of the heavy chain. Particular amino acid residues are believed to form an interface between the light and heavy chain variable domains (Clothia *et al.,* J. Mol. Biol. 186, 651-663 (1985); Novomy and Haber, Proc. Natl. Acad. Sci. USA 82, 4592-4596 [1985]).

Depending on the amino acid sequence of the constant region of their heavy chains, immunoglobulins can be assigned to different classes. There are five major classes of immunoglobulins: IgA. IgD. IgE, IgG and IgM, and several of these may be further divided into subclasses (isotypes), e.g. IgG-1, IgG-2, IgG-3, and IgG-4; IgA-1 and IgA-2. The heavy chain constant regions that correspond to the different classes of immunoglobulins are called α, delta, epsilon, γ, and µ, respectively. The subunit structures and One-dimensional configurations of different classes of immunoglobulins are well known. IgA-1 and IgA-2 are monomeric subclasses of IgA, which usually is in the form of dimers or larger polymers. Immunocytes in the gut produce mainly polymeric IgA (also referred to poly-IgA including dimers and higher polymers). Such poly-IgA contains a disulfide-linked polypeptide called the "joining" or "J" chain, and can be transported through the glandular epithelium together with the J-containing polymeric IgM (poly-IgM), comprising five subunits.

Hybridization is preferably performed under "stringent conditions" which means (I) employing low ionic strength and high temperature for washing, for example, 0.015 sodium chloride/0.0015 M sodium citrate/0.1% sodium dodecyl sulfate at 50°C, or (2) employing during hybridization a denaturing agent, such as formamide, for example, 50% (vol/vol) formamide with 0.1% bovine serum albumin/0.1% Ficoll/0.1% polyvinylpyrrolidone/50nM sodium phosphate buffer at pH 6.5 with 750 mM sodium chloride, 75 mM sodium citrate at 42°C. Another example is use of 50% formamide, 5 x SSC (0.75 M NaCl, 0.075 M sodium citrate), 50 mM sodium phosphate (pH 6/8), 0.1% sodium pyrophosphate, 5 x Denhardt's solution, sonicated salmon sperm DNA (50 µg/ml), 0.1% SDS, and 10% dextran sulfate at 42°C, with washes at 42°C in 0.2 x SSC and 0.1% SDS.

### B. OB protein-immunoglobulin chimeras (immunoadhesins)

Immunoadhesins are chimeric antibody-like molecules that combine the functional domain(s) of a binding protein (usually a receptor, a cell-adhesionmolecule or a ligand) with the an immunoglobulin sequence. The most common example of this type of fusion protein combines the hinge and Fc regions of an immunoglobulin (1g) with domains of a cell-surface receptor that recognizes a specific ligand. This type of molecule is called an "immunoadhesin", because it combines "immune" and "adhesion" functions; other frequently used names are "Ig-chimera", "Ig-" or "Fc-fusion protein", or "receptor-globulin."

To date, more than fifty immunoadhesins have been reported in the art. Immunoadhesins reported in the literature include, for example, fusions of the T cell receptor (Gascoigne *et al.,* Proc. Natl. Acad. Sci. USA 84, 2936-2940 [1987]); CD4 (Capon *et al.,* Nature 337, 525-531 [1989]; Traunecker *et al.,* Nature 339, 68-70 [1989];Zettmeissl *et al.,* DNA Cell Biol. USA 9, 347-353 [1990]; Byrn *et al.,* Nature 344, 667-670 [1990]); L-selectin (horning receptor) (Watson *et al.,* J. Cell. Biol. 110, 2221-2229 [1990]; Watson *et al.,* Nature 349, 164-167 [1991]); E-selectin [Mulligan *et al.,* J.Immunol. 151, 6410-17[1993]; Jacob *et al,* Biochemistry 34, 1210-1217 [1995]); P-selectin(Mulligan *et al*., *supra;* Hollenbaugh *et al.*, Biochemistry 34, 5678-84 [1995]); ICAM-1 (Stauton *et al.,* J.Exp. Med. 176, 1471-1476 [1992]; Martin *et al.,* J. Virol. 67, 3561-68 [1993]; Roep *et al.,* Lancet 343, 1590-93 [1994]); ICAM-2 (Damle *et al., J.* Immunol. 148, 665-71 [1992]); ICAM-3 (Holness *et al.,* J*.* Biol. Chem. 270, 877-84 [1995]); LFA-3 (Kanner *et al.,* J. Immunol. 148, 2-23-29 [1992]); L1 glycoprotein (Doherty *et al.,* Neuron 14, 57-66 [1995]); TNF-R1 (Ashkenazi *et al.,* Proc. Natl. Acad. Sci USA 88, 10535-539[1991]; Lesslauer *et al.,* Eur. J. Immunol. 21, 2883-86 [1991]; Peppel *et al.,* J. Exp. Med. 174, 1483-1489 [1991]); TNF-R2 (Zack *et al.,* Proc. Natl. Acad, Sci. USA 90, 2335-39 [1993]; Wooley *et al.,*J. Immunol. 151, 6602-07 (1993]); CD44 (Aruffo *et al.,* Cell 61, 1303-1313 (1990)]; CD28 and B7 (Linsley *et al.,* J. Exp. Med. 173*,* 721-730 (1991)]; CTLA-4 [Lisley *et al.,* J. Exp. Med. 174, 561-569 (1991)]; CD22 [Stamenkovic *et al.,* Cell 66. 1133-1144 (1991)]; NP receptors [Bennett *et al,* J. Biol. Chem. 266, 23060-23067 (1991)]; IgE receptor α [Ridgway and German. J. Cell. Biol. 115, abstr. 1448 (1991)]; HGF receptor [Mark, M.R. *et al.,* 1992, J. Biol, Chem. submitted]; IFN-γR α- and β-chain [Marsters *et al.,* Proc. Natl. Acad. Sci. USA 92, 5401-05 [1995]); trk-A, -B, and -C (Shelton *et al.,* J. Neurosci. 15, 477-91 [1995]); IL-2 (Landolfi, J. Immunol 146, 915-19 [1991]); IL-10 (Zheng *et al.,* J. Immunol. 154, 5590-5600 [1995]).

The simplest and most straightforward immunoadhesin design combines the binding region(s) of the 'adhesin' protein with the hinge and Fc regions of an immunoglobulinheavy chain. Ordinarily, when preparing the OB-immunoglobulinchimeras of the present invention, nucleic acid encoding the desired OB polypeptide will be fused C-terminally to nucleic acid encoding the N-tenminus of an immunoglobulin constant domain sequence, however N-terminal fusions are also possible. Typically, in such fusions the encoded chimeric polypeptide will retain at least functionally active hinge, CH2 and CH3 domains of the constant region of an immunoglobulin heavy chain. Fusions are also made to the C-terminus of the Fc portion of a constant domain. or immediately N-terminal to the CH1 of the heavy chain or the corresponding region of the light chain. The precise site at which the fusion is made is not critical; particular sites are well known and may be selected in order to optimize the biological activity, secretion or binding characteristics of the OB-immunoglobulin chimeras.

In a preferred embodiment, the sequence of a native, mature OB polypeptide, is fused to the N-terminus of the C-terminal portion of an antibody (in particular the Fc domain), containing the effector functions of an immunoglobulin, e.g. IgG-1. It is possible to fuse the entire heavy chain constant region to the OB sequence. However, more preferably, a sequence beginning in the hinge region just upstream of the papain cleavage site (which defines IgG Fc chemically; residue 216, taking the first residue of heavy chain constant region to be 114 [Kobet *et al., supra*], or analogous sites of other immunoglobulins) is used in the fusion. In a particularly preferred embodiment, the OB polypeptide sequence is fused to the hinge region and CH2 and CH3 or CH1, hinge, CH2 and CH3 domains of an IgG-1, IgG-2, or IgG-3 heavy chain. The precise site at which the fusion is made is not critical, and the optimal site can be determined by routine experimentation.

In some embodiments, the OB-immunoglobulinchimeras are assembled as multimers, and particularly as homo-dimers or -tetramers (WO 91 /08298). Generally, these assembled immunoglobulins will have known unit structures. A basic four chain structural unit is the form in which IgG, IgD, and IgE exist. A four unit is repeated in the higher molecular weight immunoglobulins;IgM generally exists as a pentamerof basic four units held togetherby disulfide bonds. IgA globulin,and occasionally IgG globulin, may also exist in multimeric form in serum. In the case of multimer, each four unit may be the same or different.

Various exemplary assembled OB-immunoglobulinchimeras within the scope herein are schematically diagrammed below:
(a) AC_{L}-AC_{L};
(b) AC_{H}-[AC_{H}, AC_{L}-AC_{H}, AC_{L}-V_{H}C_{H}, or V_{L}C_{L}-AC_{H}];
(c) AC_{L}-AC_{H}-[AC_{L}-AC_{H}, AC_{L}-V_{H}C_{H}, V_{L}C_{L}-AC_{H}, or V_{L}C_{L}-V_{H}C_{H}];
(d) AC_{L}-V_{H}C_{H}-[AC_{H}, or AC_{L}-V_{H}C_{H}, or V_{L}C_{L}-AC_{H}];
(e) V_{L}C_{L}-AC_{H}-[AC_{L}-V_{H}C_{H}, or V_{L}C_{L}-AC_{H}]; and
(f) [A-Y]ₙ-[V_{L}C_{L}-V_{H}C_{H}]₂,
wherein
each A represents identical or different OB polypeptide amino acid sequences;
V_{L} is an immunoglobulin light chain variable domain;
V_{H} is an immunoglobulin heavy chain variable domain;
C_{L} is an immunoglobulin light chain constant domain;
C_{H} is an immunoglobulin heavy chain constant domain;
n is an integer greater than 1;
Y designates the residue of a covalent cross-linking agent.

In the interests of brevity, the foregoing structures only show key features; they do not indicate joining (J) or other domains of the immunoglobulins, nor are disulfide bonds shown. However, where such domains are required for binding activity, they shall be constructedas being present in the ordinary locations which they occupy in the immunoglobulin molecules.

Alternatively, the OB amino acid sequences can be inserted between immunoglobulinheavy chain and light chain sequences such that an immunoglobulin comprising a chimeric heavy chain is obtained. In this embodiment, the OB polypeptide sequences are fused to the 3' end of an immunoglobulin heavy chain in each arm of an immunoglobulin, either between the hinge and the CH2 domain, or between the CH2 and CH3 domains. Similar constructs have been reported by Hoogenboom, H. R. *et al.,* Mol. Immunol. 28, 1027-1037 (1991).

Although the presence of an immunoglobulin light chain is not required in the immunoadhesins of the present invention, an immunoglobulin light chain might be present either covalently associated to an OB protein-immunoglobulin heavy chain fusion polypeptide, or directly fused to the OB polypeptide. In the former case, DNA encoding an immunoglobulin light chain is typically coexpressed with the DNA encoding the OB-immunoglobulinheavy chain fusion protein. Upon secretion, the hybrid heavy chain and the light chain will be covalently associated to provide an immunoglobulin-like structure comprising two disulfide-linked immunoglobulinheavy chain-light chain pairs. Method suitable for the preparation of such structures are, for example, disclosed in U.S. Patent No. 4,816,567 issued 28 March 1989.

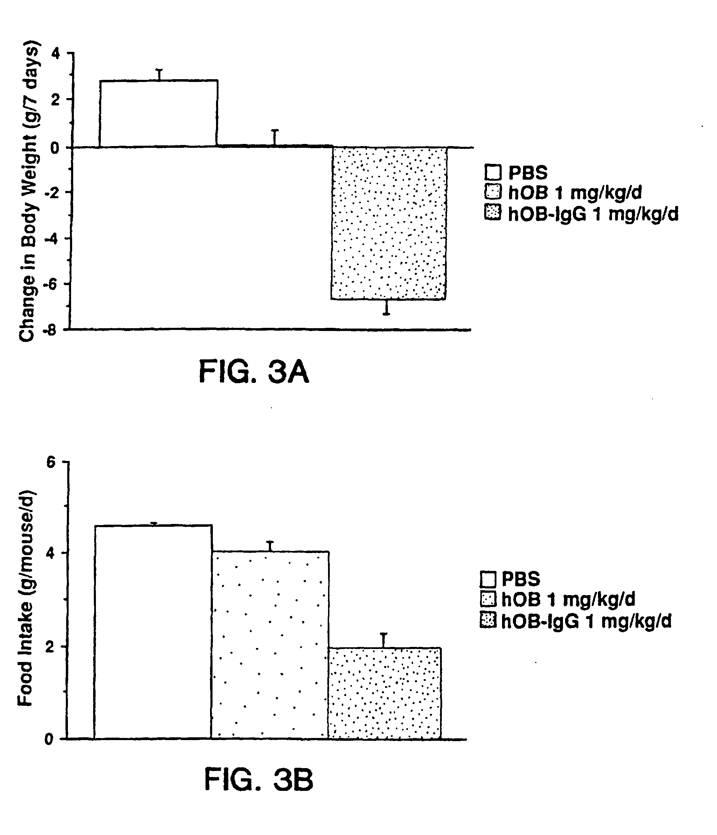

In a preferred embodiment, the immunoglobulin sequences used in the construction of the immunoadhesins of the present invention are from an IgG immunoglobulin heavy chain constant domain. For human immunoadhesins,the use of human IgG-1 and IgG-3 immunoglobulin sequences is preferred. A major advantage of using IgG-1 is that IgG-1 immunoadhesins can be purified efficiently on immobilized protein A. In contrast, purification of IgG-3 requires protein G, a significantly less versatile medium. However, other structural and functional properties of immunoglobulins should be considered when choosing the Ig fusion partner for a particular immunoadhesin construction. For example, the IgG-3 hinge is longer and more flexible, so it can accommodate larger 'adhesin' domains that may not fold or function properly when fused to IgG- 1. Possible IgG-based immunoadhesin structures are shown in Fig. 3a-c. While IgG immunoadhesinsare typically mono- or bivalent, other Ig subtypes like IgA and IgM may give rise to dimeric or pentameric structures, respectively, of the basic Ig homodimerunit. A typical IgM-based multimeric immunoadhesin is illustrated in Figure 3d. Multimeric immunoadhesins are advantageous in that they can bind their respective targets with greater avidity than their IgG-based counterparts. Reported examples of such structures are CD4-IgM (Traunecker *et al., supra);* ICAM-IgM (Martin *et al.,* J. Virol. 67, 3561-68 [1993]); and CD2-IgM (Arulanandam *et al.,* J. Exp. Med. 177, 1439-50 [1993]).

For OB-Ig immunoadhesins, which are designed for *in vivo* application, the pharmacokineticproperties and the effector functions specified by the Fc region are important as well. Although IgG-1, IgG-2 and IgG-4 all have *in vivo* half-lives of 21 days, their relative potencies at activating the complement system are different. IgG-4 does not activate complement, and IgG-2 is significantly weaker at complement activation than IgG-1. Moreover, unlike IgG-1, IgG-2 does not bind to Fc receptors on mononuclear cells or neutrophils. While IgG-3 is optimal for complement activation, its *in vivo* half-life is approximately one third of the other IgG isotypes. Another important consideration for immunoadhesinsdesigned to be used as human therapeutics is the number of allotypic variants of the particular isotype. In general, IgG isotypes with fewer serologically-defined allotypes are preferred. For example, IgG-1 has only four serologically-defined allotypic sites, two of which (G 1m and 2) are located in the Fc region; and one of these sites Glm1, is non-immunogenic. In contrast, there are 12 serologically-defined allotypes in IgG-3, all of which are in the Fc region; only three of these sites (G3m5, 11 and 21) have one allotype which is nonimmunogenic. Thus, the potential immunogenicity of a γ3 immunoadhesin is greater than that of a γ 1 immunoadhesin.

In designing the OB-Ig immunoadhesins of the present invention regions that are not required for receptor binding, the structural integrity (e.g. proper folding) and/or biological activity of the molecule, may be deleted. In such structures, it is important to place the fusion junction at residues that are located between domains, to avoid misfolding. With respect to the parental immunoglobulin, a useful joining point is just upstream of the cysteines of the hinge that form the disulfide bonds between the two heavy chains. In a frequently used design, the codon for the C-terminal residue of the "adhesin" (OB) part of the molecule is placed directly upstream of the codons for the sequence DKTHTCPPCP of the IgG1 hinge region.

OB-Ig immunoadhesinsare most conveniently constructed by fusing the cDNA sequence encoding the OB portion in-frame to an Ig cDNA sequence. However, fusion to genomic Ig fragments can also be used (see, e.g. Gascoigne *et al.,* Proc. Natl. Acad. Sci. USA 84, 2936-2940 [1987]; Aruffo *et al.,* Cell 61, 1303-1313 [1990]; Stamenkovic *et al.,* Cell 66*,*1133-1144 [1991]). The latter type of fusion requires the presence of Ig regulatory sequences for expression. cDNAs encoding IgG heavy-chain constant regions can be isolated based on published sequence from cDNA libraries derived from spleen or peripheral blood lymphocytes, by hybridization or by polymerase chain reaction (PCR) techniques. Murine OB cDNA can, for example, be obtained by PCR from a mouse adipose tissue cDNA library (Clontech), using primers designed based on the sequence of Zhang *et al.* Human OB cDNA can be obtained in a similar manner. Alternatively, the mouse OB gene can be used as a probe to isolate human adipose tissue cDNA clones (Clontech), e.g. from a λgtII library, as described by Zhang *et al.* The cDNAs encoding the 'adhesin' and the Ig parts of the immunoadhesin are inserted in tandem into a plasmid vector that directs efficient expression in the chosen host cells. For expression in mammalian cells pRK5-based vectors (Schall *et al*, Cell 61, 361 -370 [1990]), pRK7-vectors and CDM8-based vectors (Seed, Nature 329, 840 [1989]) are preferred. (pRK7 is identical to pRK5 except that the order of the endonuclease restriction sites in the polylinker region between ClaI and HindIII is reversed. See U.S. Patent No. 5,108,901 issued 28 April 1992.). The exact junction can be created by removing the extra sequences between the designed junction codons using oligonucleotide-directed deletional mutagenesis (Zoller and Smith, Nucleic Acids Res. 10, 6487 [1982]; Capon *et al*., Nature 337, 525-531 [1989]). Synthetic oligonucleotidescan be used, in which each half is complementary to the sequence on either side of the desired junction; ideally, these are 36 to 48-mers. Altenatively, PCR technique can be used to join the two parts of the molecule in-frame with an appropriate vector.

Immunoadhesinscan be expressed efficiently in a variety of host cells, including myeloma cell lines, Chinese Hamster ovary (CHO) cells, monkey COS cells, human embryonic kidney 293 cells, and baculovirus infected insect cells. In these systems, the immunoadhesin polypeptides are assembled and secreted into the cell culture medium. Yeasts, e.g. Saccharomyces cerevisiae, Pichia pastoris, etc., and bacterial cells, preferably E. coli, can also be used as hosts. The OB-immunoglobulin chimeras can be expressed in yeast, for example, similarly to the process described for the expression of the OB proteins by Leiber *et al.,* Crit. Res. Food Sci. Nutr, 33, 351 (1993); Friedman and Leibel, cell 69, 217 (1992); and Beavis and Chait, Proc. Natl. Acad. Sci. USA 87, 6873 (1990). Thus, the coding sequences can be subcloned into a yeast plasmid, such as the yeast expression plasmid pPIC.9 (Invitrogen). This vector directs secretion of heterologous proteins from the yeast into the culture media. According to Halaas *et al., supra,* expression of mouse and human OB genes in Saccharomyces cerevisiae transformed with this vector yields a secreted 16-kD protein, which is an unprocessed OB protein lacking the signal sequence. Expression of the mouse or human OB-immunoglobulin chimeras in E. coli can, for example, be performed on the analogy of the procedure described by Halaas *et al., supra.* The coding sequences of mouse and human OB-immunoglobulin chimeras can be subcloned into the PET15b expression vector (Novagen) and expressed in E. coli (BL21 (DE3)pIYsS) through use of the T7 E. coli RNA polymerase system. Alternatively, the fusion protein can be expressed in E. coli by inserting the coding sequence in frame with the secretion sequence of the E. coli heat stable enterotoxin II, downstream of the E. coli alkaline phosphatase promoter (Chang *et al.,* Gene 55, 189-96 [1987]).

The choice of host cell line for the expression of OB-Ig immunoadhesins depends mainly on the expression vector. Another consideration is the amount of protein that is required. Milligram quantities often can be produced by transient transfections. For example, the adenovirus EIA-transformed 293 human embryonic kidney cell line can be transfected transiently with pRK5- and pRK7-based vectors by a modification of the calcium phosphate method to allow efficient immunoadhesin expression. This method is illustrated in the examples. CDM8-based vectors can be used to transfect COS cells by the DEAE-dextran method (Aruffo *et al.,* Cell 61*,* 1303-1313(1990); Zettmeissl *et al.,* DNA Cell Biol.(US) 9,347-353 (1990)]. If larger amounts of protein are desired, the immunoadhesin can be expressed after stable transfection of a host cell line. For example, a pRKS- or pRK7-based vector can be introduced into Chinese hamster ovary (CHO) cells in the presence of an additional plasmid encoding dihydrofolatereductase (DHFR) and conferring resistance to G418. Clones resistantto G418 can be selected in culture; these clones are grown in the presence of increasing levels of DHFR inhibitor methotrexate;clones are selected, in which the number of gene copies encoding the DHFR and immunoadhesin sequences is co-amplified. If the immunoadhesincontains a hydrophobic leader sequence at its N-terminus, it is likely to be processed and secreted by the transfected cells. The expression of immunoadhesins with more complex structuresmay require uniquely suited host cells; for example, components such as light chain or J chain may be provided by certain myeloma or hybridoma cell hosts [Gascoigne *et al.,* 1987, *supra;* Martin *et al.,* J. Virol. 67, 3561-3568 (1993)].

The expression of immunoadhesins with more complex oligomeric structures may require uniquely suited host cells; for example, components such as light chain or J chain may be provided by certain myeloma or hybridoma cell hosts (Gascoigne *et al., supra;* Martin *et al.,* J. Immunol. 67, 3561-68 [1993]).

Immunoadhesinscan be convenientlypurified by affinity chromatography. The suitability of protein A as an affinity ligand depends on the species and isotype of the immunoglobulin Fc domain that is used in the chimera. Protein A can be used to purify immunoadhesinsthat are based on human γ1, γ2, or γ4 heavy chains [Lindmark *et al., J.* Immunol. Meth. 62, 1-13 (1983)]. Protein G is recommended for all mouse isotypes and for human γ3 [Guss *et al.,* EMBO J. 5. 15671575 (1986)]. The matrix to which the affinity ligand is attached is most often agarose, but other matrices are available. Mechanically stable matrices such as controlled pore glass or poly(styrenedivinyl)benzene allow for faster flow rates and shorter processing times than can be achieved with agarose. The conditions for binding an immunoadhesinto the protein A or G affinity column are dictated entirely by the characteristics of the Fc domain; that is, its species and isotype. Generally, when the proper ligand is chosen, efficient binding occurs directly from unconditioned culture fluid. One distinguishing feature of immunoadhesins is that, for human γ1 molecules, the binding capacity for protein A is somewhat diminishedrelative to an antibody of the same Fc type. Bound immunoadhesin can be efficiently eluted either at acidic pH (at or above 3.0), or in a neutral pH buffer containing a mildly chaotropic salt. This affinity chromatography step can result in an immunoadhesin preparation that is >95% pure.

Other methods known in the art can be used in place of, or in addition to, affinity chromatography on protein A or G to purify immunoadhesins. Immunoadhesins behave similarly to antibodies in thiophilic gel chromatography [Hutchens and Porath, Anal. Biochem. 159, 217-226 (1986)] and immobilized metal chelate chromatography [Al-Mashikhi and Makai, J. Dairy Sci. 71, 1756-1763 (1988)]. In contrast to antibodies, however, their behavior on ion exchange columns is dictated not only by their isoelectric points, but also by a charge dipole that may exist in the molecules due to their chimeric nature. Microheterogeneity of charge can also be a factor for inununoadhesins in which the adhesin portion of the molecule is glycosylated and contains sialic acid. A specific purification protocol is described in the examples.

Results with the numerous immunoadhesinsproduced so far show that the fusion of the adhesin portion to an Fc region usually does not perturb the folding of the individual domains. Both the adhesin and the immunoglobulin regions appear to fold correctly, and the Fc portion retins many of the effector functions that are characteristic of antibodies, such as binding to Fc receptors.

Methods generally applicable for the construction, expression and purification of immunoadhesins are described, for example, in U.S. Patent Nos. 5,225,538 (issued 6 July 1993) and 5,455,165 (issued 30 October 1995), the disclosures of which are hereby expressly incorporated by reference. Immunoadhesin construction, expression, purification and various immunoadhesins designs are also described in the review articles by Ashkenazi and Chamow, Methods in Enzymology 8, 104-115 (1995), and Peach and Linsley, Methods in Enzymology 8, 116-123 (1995), the disclosures of which, along with the references cited therein, is hereby expressly incorporated by reference.

### C. Other long half-life OB derivatives

Other derivatives of the OB proteins, which possess a longer half-life than the native molecules comprise the OB protein or an OB-immunoglobulinchimera, **c**ovalently bonded to a nonproteinaceouspolymer. The nonproteinaceouspolymer ordinarily is a hydrophilic synthetic polymer, i.e., a polymer not otherwise found in nature. However, polymers which exist in nature and are produced by recombinant or *in vitro* methods are useful, as are polymers which are isolated from native sources. Hydrophilic polyvinyl polymers fall within the scope of this invention, e.g. polyvinylalcohol and polyvinylpyrrolidone. Particularly useful are polyalkylene ethers such as polyethyleneglycol (PEG); polyelkylenessuch as polyoxyethylene, polyoxypropylene,and block copolymers of polyoxyethylene and polyoxypropylene (Pluronics); polymethacrylates; carbomers; branched or unbranched polysaccharides which comprise the saccharide monomers D-mannose, D- and L-galactose, fucose, fructose, D-xylose, L-arabinose, D-glucuronic acid, sialic acid, D-galacturonicacid, D-mannuronic acid (e.g. polymannuronicacid, or alginic acid), D-glucosamine, D-galactosamine, D-glucose and neuraminic acid including homopolysaccharides and heteropolysaccharides such as lactose, amylopectin, starch, hydroxyethyl starch, amylose, dextrane sulfate, dextran, dextrins, glycogen, or the polysaccharide subunit of acid mucopolysaccharides,e.g. hyaluronic acid; polymers of sugar alcohols such as polysorbitol and polymannitol; heparin or heparon. The polymer prior to cross-linking need not be, but preferably is, water soluble, but the final conjugate must be water soluble. In addition, the polymer should not be highly immunogenic in the conjugate form, nor should it possess viscosity that is incompatible with intravenous infusion or injection if it is intended to be administered by such routes.

Preferably the polymer contains only a single group which is reactive. This helps to avoid cross-linking of protein molecules. However, it is within the scope herein to optimize reaction conditions to reduce cross-linking, or to purify the reaction products through gel filtration or chromatographic sieves to recover substantially homogenous derivatives.

The molecular weight of the polymer may desirably range from about 100 to 500,000, and preferably is from about 1,000 to 20,000. The molecular weight chosen will depend upon the nature of the polymer and the degree of substitution. In general, the greater the hydrophilicity of the polymer and the greater the degree of substitution, the lower the molecular weight that can be employed. Optimal molecular weights will be determined by routine experimentation.

The polymer generally is covalently linked to the OB protein or to the OB-immunoglobulin chimeras though a multifunctionalcrosslinkingagent which reacts with the polymer and one or more amino acid or sugar residues of the OB protein or OB-immunoglobulinchimera to be linked. However, it is within the scope of the invention to directly crosslink the polymer by reacting a derivatized polymer with the hybrid, or via versa.

The covalent crosslinking site on the OB protein or OB-Ig includes the N-terminal amino group and epsilon amino groups found on lysine residues, as well as other amino, imino, carboxyl, sulfhydryl, hydroxyl or other hydrophilic groups. The polymer may be covalently bonded directly to the hybrid without the use of a multifunctional (ordinarily bifunctional) crosslinking agent. Covalent binding to amino groups is accomplished by known chemistries based upon cyanuric chloride, carbonyl diimidazole, aldehyde reactive groups (PEG alkoxide plus diethyl acetal of bromoacetaldehyde;PEG plus DMSO and acetic anhydride, or PEG chloride plus the phenoxide of 4-hydroxybenzaldehyde, succinimidyl active esters, activated dithiocarbonate PEG, 2,4,5-trichlorophenylcloroformate or P-nitrophenylcloroforntate activated PEG.) Carboxyl groups are derivatized by coupling PEG-amine using carbodiimide.

Polymers are conjugated to oligosaccharide groups by oxidation using chemicals, e.g. metaperiodate, or enzymes, e.g. glucose or galactose oxidase, (either of which produces the aldehyde derivative of the carbohydrate), followed by reaction with hydrazide or amino derivatized polymers, in the same fashion as is described by Heitzmann *et al.,* P.N.A.S., 71, 3537-41 (1974) or Bayer *et al.,* Methods in Enzymology 62, 310 (1979), for the labeling of oligosaccharideswith biotin or avidin. Further, other chemical or enzymatic methods which have been used heretofore to link oligosaccharides are particularly advantageous because, in general, there are fewer substitutions than amino acid sites for derivatitation, and the oligosaccharide products thus will be more homogenous. The oligosaccharide substituents also are optionally modified by enzyme digestion to remove sugars, e.g. by neuraminidase digestion, prior to polymer derivatization.

The polymer will bear a group which is directly reactive with an amino acid side chain, or the N- or C-tenninus of the polypeptide linked, or which is reactive with the multifunctional cross-linking agent. In general, polymers bearing such reactive groups are known for the preparation of immobilized proteins, In order to use such chemistries here, one should employ a water soluble polymer otherwise derivatized in the same fashion as insoluble polymers heretofore employed for protein immobilization. Cyanogen bromide activation is a particularly useful procedure to employ in crosslinking polysaccharides.

"Water soluble" in reference to the starting polymer means that the polymer or its reactive intermediate used for conjugation is sufficiently water soluble to participate in a derivatization reaction.

"Water soluble" in reference to the polymer conjugate means that the conjugate is soluble in physiological fluids such as blood.

The degree of substitution with such a polymer will vary depending upon the number of reactive sites on the protein, whether all or a fragment of the protein is used, whether the protein is a fusion with a heterologous protein (e.g. an OB-immunoglobulin chimera), the molecular weight, hydrophilicity and other characteristics of the polymer, and the particular protein derivatization sites chosen. In general, the conjugate contains about from 1 to 10 polymer molecules, while any heterologous sequence may be substituted with an essentially unlimited number of polymer molecules so long as the desired activity is not significantly adversely affected. The optimal degree of cross-linking is easily determined by an experimental matrix in which the time, temperature and other reaction conditions are varied to change the degree of substitution, after which the ability of the conjugates to function in the desired fashion is determined.

The polymer, e.g. PEG, is cross-linked by a wide variety of methods known *per se* for the covalent modificationof proteins with nonproteinaceouspolymers such as PEG. Certain of these methods, however, are not preferred for the purposes herein. Cyanuronic chloride chemistry leads to many side reactions, including protein cross-linking. In addition, it may be particularly likely to lead to inactivation of proteins containing sulfhydryl groups. Carbonyl diimidazole chemistry (Beauchamp *et al.,* Anal Biochem. 131, 25-33 [1983]) requires high pH (>8.5), which can inactivate proteins. Moreover, since the "activated PEG" intermediate can react with water, a very large molar excess of "activated PEG" over protein is required. The high concentrations of PEG required for the carbonyldiimidazole chemistry also led to problems in purification, as both gel filtration chromatography and hydrophilic interaction chromatography are adversely affected. In addition, the high concentrations of "activated PEG" may precipitate protein, a problem that *per se* has been noted previously (Davis, U.S. Patent No. 4,179,337). On the other hand, aldehyde chemistry (Royer, U.S. Patent No. 4,002,531) is more efficient since it requires only a 40-fold molar excess of PEG and a 1-2 hr incubation. However, the manganese dioxide suggested by Royer for preparation of the PEG aldehyde is problematic "because of the pronounced tendency of PEG to form complexes with metal-based oxidizing agents" (Harris *et al.* J. Polym. Sci, Polym. Chem. Ed. 22, 341-52 [1984]). The use of a Moffatt oxidation, utilizing DMSO and acetic anhydride, obviates this problem. In addition, the sodium borohydride suggested by Royer must be used at high pH and has a significant tendency to reduce disulfide bonds. In contrast, sodium cyanoborohydride, which is effective at neutral pH and has very little tendency to reduce disulfide bonds is preferred.

Functionalized PEG polymers to modify the OB protein or OB-Ig chimeras of the present invention are available from Shearwater Polymers, Inc. (Huntsville, AL). Such commercially available PEG derivatives include, but are not limited to, amino-PEG, PEG amino acid esters, PEG-hydrazide, PEG-thiol, PEG-succinate, carboxymethylated PEG, PEG-propionicacid, PEG amino acids. PEG succinimidyisuccinate, PEG succinimidyl propionate, succinimidyl ester of carboxymethylatedPEG, succinimidyl carbonate of PEG, succinimidyl esters of amino acid PEGs, PEG-oxycarbonylimidazole, PEG-nitrophenyl carbonate, PEG tresylate, PEG-glycidyl ether. PEG-aldehyde,PEG vinylsulfone,PEG-maleimide,PEG-orthopyridyl-disulfide, heterofunctional PEGs, PEG vinyl derivatives, PEG silanes, and PEG phospholides. The reaction conditions for coupling these PEG derivatives will vary depending on the protein, the desired degree of PEGylation, and the PEG derivative utilized. Some factors involved in the choice of PEG derivatives include: the desired point of attachment(lysine or cysteine), hydrolytic stability and reactivity of the derivatives, stability, toxicity and antigenicity of the linkeage, suitabilityfor analysis, etc. Specific instructions for the use of any particular derivative are available from the manufacturer.

The long half-life conjugates of this invention are separated from the unreacted starting materials by gel filtration. Heterologous species of the conjugates are purified from one another in the same fashion. The polymer also may be water-insoluble, as a hydrophilic gel.

The conjugates may also be purified by ion-exchange chromatography. The chemistry of many of the electrophilicallyactivated PEG's results in a reduction of amino group charge of the PEGylated product. Thus, high resolution ion exchange chromatography can be used to separate the free and conjugated proteins, and to resolve species with different levels of PEGylation. In fact, the resolution of different species (e.g. containing one or two PEG residues) is also possible due to the difference in the ionic properties of the unreacted amino acids.

### D. The use of the OB-immunoglobulin chimeras and other long half-life derivatives

The OB-immunoglobulinchimeras and other long half-life OB derivatives of the present invention are useful for weight reduction, and specifically, in the treatment of obesity and other disorders associated with the abnormal expression or function of the OB gene. Our studies indicate that the OB-immunoglobulin chimeras and other long half-life OB derivatives,e.g. PEGylated OB, reduce the food intake and increase the energy use of animals treated, and are therefore very effective in reducing the weight of both obese and normal subjects. For testing purposes, the molecules of the present invention may be dissolved in phosphate-buffered saline (PBS) (pH 7.4), and administered by intravenous or subcutaneous injection, or infusion.

The long acting OB-derivatives of the present invention may further be used to treat other metabolic disorders such as diabetes and bulimia. The OB protein has been shown to reduce insulin levels in animals, and could be useful to reduce excessive levels of insulin in human patients. The reduction of insulin levels in obese or non-obese patients (e.g. Type or II diabetics) could restore or improve the insulin-sensitivity of such patients.

In addition, the long half-life OB-derivatives can be used for the treatment of kidney ailments, hypertension, and lung disfunctions, such as emphysema. The OB protein might also cause a mitogenic response in receptor-bearing tissues, acting as a growth factor for these cells.

Therapeutic formulations of the present invention are prepared for storage by mixing the active ingredient having the desired degree of purity with optional physiologically acceptable carriers, excipients or stabilizers (Reminglon's Pharmaceutical Sciences 16th edition, Osol, A. Ed. (1980)), in the form of lyophilized formulations or aqueous solutions. Acceptable carriers, excipients or stabilizers are nontoxic to recipients at the dosages and concentrations employed, and include buffers such as phosphate, citrate and other organic acids; antioxidants including ascorbic acid; low molecular weight (less than about 10 residues) polypeptides; proteins, such as serum albumin, gelatin or immunoglobulins;hydrophilic polymers such as polyvinylpyrrolidone,amino acids such as glycine, glutamine, asparagine, arginine or lysine; monosaccharides, disaccharides and other carbohydrates including glucose, mannose, or dextrins; chelating agents such as EDTA; sugar alcohols such as mannitol or sorbitol; salt-forming counterions such as sodium; and/or nonionic surfactants such as Tween, Pluronics or PEG.

The active ingredientsmay also be entrapped in microcapsules prepared, for example, by coacervation techniques or by interfacial polymerization, for example, hydroxymethylcellulose or gelatin-microcapsules and poly-(methylmethacylate) microcapsules, respectively), in colloidal drug delivery systems (for example, liposomes, albumin microspheres, microemulsions, nano-particles and nanocapsules) or in macroemulsions. Such techniques are disclosed in Remington's Pharmaceutical Sciences, *supra.*

The formulations to be used for *in vivo* administration must be sterile. This is readily accomplished by filtration through sterile filtration membranes, prior to or following lyophilization and reconstitution.

Therapeuticcompositions herein generally are placed into a container having a sterile access port, for example, an intravenous solution bag or vial having a stopper pierceable by a hypodermic injection needle.

The route of administration is in accord with known methods, e.g. injection or infusion by intravenous, intraperitoneal,etc. routes. Sustained released formulations are also foreseen. Suitable examples of sustained release preparations include semipermeable polymer matrices in the form of shaped articles, e.g. films, or microcapsules. Sustained release matrices include polyesters, hydrogels, polylactides (U.S. Patent 3,773,919, EP 58,481), copolymers of L-glutamic acid and gamma ethyl-L-glutamate (U. Sidman et al., 1983, "Biopolymers" 22 (1): 547-556), poly (2-hydroxyethyl-methacrylate) (R. Langer, et al., 1981, "J. Biomed. Mater. Res." 15: 167-277 and R. Langer, 1982, Chem. Tech." 12: 98-105), ethylene vinyl acetate (R. Langer et al., Id.) or poly-D-(-)-3-hydroxybutyric acid (EP 133,988A). Sustained release compositions also include liposomes. Liposomes containing molecule within the scope of the present invention are prepared by methods known per se: DE 3,218,121 A; Epstein et al., 1985, "Proc. Natl. Acad. Sci. USA" 82: 3688-3692; Hwang et al., 1980, "Proc. Natl. Acad. Sci. USA" 77: 4030-4034; EP 52322A; EP 36676A; EP 88046A; EP 143949A; EP 142641A; Japanese patent application 83-118008;U.S. patents 4,485,045 and 4,544,545; and EP 102,324A. Ordinarily the liposomesare of the small (about 200-800 Angstroms) unitamelartype in which the lipid content is greater than about 30 mol. % cholesterol, the selected proportion being adjusted for the optimal therapy.

An effective amount of a molecule of the present invention to be employed therapeuticatly will depend, for example, upon the therapeutic objectives, the route of administration, and the condition of the patient. Accordingly, it will be necessary for the therapist to titer the dosage and modify the route of administration as required to obtain the optimal therapeutic effect. A typical daily dosage might range from about 1 µg/kg to up to 100 mg/kg or more, depending on the factors mentioned above. Typically, the clinician will administer a molecule of the present invention until a dosage is reached that provides the required biological effect. The progress of this therapy is easily monitored by conventional assay techniques. If the purpose of the treatment is weight reduction, the therapy is normally continued until a desired body weight is reached.

Non-therapeuticuses of the OB protein-immunoglobulinfusions of the present invention include their use to identify and purify OB receptors. The identificationand expression cloning of an OB receptor, using an OB protein-immunoadhesin is described in a Reference Example hereinbelow.

The invention will be further illustrated by the following non-limiting examples.

### Example 1

### Expression of OB- immunoadhesins

Using protein engineering techniques, the human OB protein was expressed as a fusion with the hinge, CH2 and CH3 domains of IgG-1. DNA constructs encoding the chimera of the human OB protein and IgG-1 Fc domains were made with the Fc region clones of human IgG-1. Human OB cDNA was obtained by PCR from human fat cell dscDNA (Clontech Buick-ClonecDNA product). The source of the IgG-1 cDNA was the plasmid pBSSK-CH₂CH₃. The chimera contained the coding sequence of the full length OB protein (amino acids 1-167 in Figure 5) and human IgG-1 sequences beginning at aspartic acid 216 (taking amino acid 114 as the first residue of the heavy chain constant region (Kabat *et al.,* Sequences of Proteins of Immunological Interest 4th ed. [1987]), which is the first residue of the IgG-1 hinge after the cysteine residue involved in heavy-light chain bonding, and ending with residues 441 to include the CH2 and CH3 Fc domains of IgG-1. There was an insertofcodons for three amino acids (GlyValThr) between the OB and IgG-1 coding sequences. If necessary, this short linker sequence can easily be deleted, for example by site directed deletion mutagenesis, to create an exact junction between the coding sequences of the OB protein and the IgG-1 hinge region. The coding sequence of the OB-IgG-1 immunoadhesin was subcloned into the pRK5-based vector pRK5tk-neo which contains a neomycine selectablemarker, for transientexpression in 293 cells using the calcium phosphate technique (Suva *et al,* Science 237, 893-896 [1987]). 293 cells were cultured in HAM's : Low Glucose DMEM medium (50:50), containing 10% FBS and 2 mM L-Gln. For purification of OB-IgG-1 chimeras, cells were changed to serum free production medium PS24 the day after transfectionand media collected after three days. The culture media was filtered.

The filtered 293 cell supetnatant(400 ml) containing recombinant human OB-IgG-1 was made 1 mM in phenylmethylsulfonylfluoride and 2 µg/ml in aprotinin. This material was loaded at 4 °C onto a 1 x 4.5 cm Protein A agarose column (Pierce catalog # 20365) equilibrated in 100 mM HEPES pH 8. The flow rate was 75 ml/h. Once the sample was loaded, the column was washed with equilibration buffer until the A₂₈₀ reached baseline. The OB-IgG-1 protein was eluted with 3.5 M MgCl₂ + 2% glycerol (unbuffered) at a flow rate of 15 ml/h. The eluate was collected with occasional mixing into 10 ml of 100 mM HEPES pH 8 to reduce the MgCl₂ concentration by approximately one-half and to raise the pH. The eluted protein was then dialyzed into phosphate buffered saline, concentrated, sterile filtered and stored either at 4°C or frozen at -70 °C. The OB-IgG-1 immunoadhesin prepared by this method is estimated by SDS-PAGE to be greater than 90% pure.

### Example 2

### Animal studies

### A. Material and Methods

OB protein Production - Murine OB cDNA was obtained by PCR from an adipocyte cDNA library using primers based on the sequence of Zhang *et al., supra.* Mature OB protein (amino acids 22-167) was expressed in E. coli by inserting the OB coding sequence in frame with the secretion sequence of the E. coli heat-stable enterotoxin II, downstream of the E. coli alkaline phosphatasepromoter. Chang *et al.,* Gene 55, 189-96 (1987). After cell lysis, the insoluble fraction was solubilized in 8 M urea buffer pH 8.35 in the presence of 25 mM DTT. Reduced OB protein was purified by size exclusion and reverse phase HPLC, then refolded in the presence of glutathione. Refolded OB protein was purified by reverse phase HPLC and analyzed by SDS-PAGE and amino acid and mass spectrometry analyses.

Preparation of PEG-hOB - - The PEG derivatives of the human PB protein were prepared by reaction of hOB purified by reverse phase chromatographywith a succinimidyl derivative of PEG propionic acid (SPA-PEG) having a nominal molecular weight of 10 kD, which had been obtained from Shearwater Polymers, Inc. (Huntsville, AL). After purification of the hOB protein by reverse phase chromatography, an approximately 1-2 mg/ml solution of the protein in 0.1 % trifluoroacetic acid and approximately 40% acetonitrile. was diluted with 1/3 to 1/2 volume of 0.2 M borate buffer and the pH adjusted to 8.5 with NaOH. SPA-PEG was added to the reaction mixture to make 1:1 and 1:2 molar ratios of protein to SPA-PEG and the mixture was allowed to incubate at room temperature for one hour. After reaction and purification by gel electrophoresis or ion exchange chromatography, the samples were extensively dialyzed against phosphate-buffered saline and sterilized by filtration through a 0.22 micron filter. Samples were stored at 4°C. Under these conditions, the PEG-hOB resulting from the 1:1 molar ratio protein to SPA-PEG reaction consisted primarily of molecules with one 10 kD PEG attached with minor amounts of the 2 PEG-containing species. The PEG-hOB from the 1:2 molar reaction consisted of approximately equal amounts of 2 and 3 PEGs attached to hOB, as determined by SDS gel electrophoresis. In both reactions, small amounts of unreacted protein was also detected. This unreacted protein can be efficiently removed by the gel nitration or ion exchange steps as needed. The PEG derivatives of the human OB protein can also be prepared essentially following the aldehyde chemistry described in EP 372,752 published June 13, 1990.

Animal Studies -- All manipulations involving animals were reviewed and approved by Genentech's Institutional Animal Care and Use Committee. Seven to eight week-old genetically obese C57BI/6J-*ob*/*ob (ob*/*ob)* female mice were purchase from Jackson Labs (Bar Harbor, ME). Lean female mice of the same genetic background (C57BI/6) were purchased from Harlan Sprague Dawley (Hollister, CA). Mice were housed in groups 3-6 with *ad libitum* access to water and standard mouse chow (Purina 5010; Purina Mills, Richmond, IN) in a temperature-, humidity- and light-controlled (lights on at 06:00h, of at 18:00h) colony room.

Miniosmotic pumps (Alzet model 2002; Alza Corp., Palo Alto, CA) were filled with purified recombinant OB protein (100 µg/kg/day) in sterile phosphate-buffered saline (PBS) or PBS alone under sterile conditions following manufacturer's instructions and incubated overnight in sterile saline at room temperature prior to implantation into mice. Mice were anesthetized with ketamine/xylazine, and miniosmotic pumps were implanted subcutaneously in the midscapular region. Daily subcutaneous injections of purified recombinant OB protein, hOB-IgG-1 fusion protein or PBS were made into the midscapular region of conscious mice. Injections were performed within one hour of lights out. The body weight of each mouse (to the nearest 0.1 gram) and the weight of the food contained in the food bin in each cage (to the nearest 0. 1 gram) were recorded within one hour of lights out every one to two days. The data are depicted as the mean ± SEM. The number of animals is as described below and in the Figure legends.

### B. Results with continuous subcutaneous infusion of OB protein

Lean female mice were treated with murine OB protein either as a continuous subcutaneous infusion or daily subcutaneous injections. The results are shown in Figure 1. The upper chart shows that the OB protein is significantlymore effective in reducing body weight when delivered as a continuous infusion than when the same dose is delivered in the form of daily subcutaneous injections. The bottom chart shows the same difference in the ability of the OB protein to reduce adipose tissue weight.

### C. Results with the OB-IgG-1 chimera

Obese female *ob*/*ob* mice were treated with human OB protein or with the human OB-IgG-1 chimera. The data are shown in Figure 2. The data presented in the top chart demonstrate that the hOB-IgG-1 fusion protein is more potent than the native hOB protein at reducing body weight, when both proteins are administered similarly by daily subcutaneous infusion. It is noted that the increase in potency would be even more expressed, if the data were converted to molar amounts, as only about one third of the OB-IgG-1 chimera comes from the OB protein. The data further confirm the previous observation that continuous subcutaneous infusion (pump) or the hOB protein is more effective than daily subcutaneous injections (inj) at reducing body weight.

The data shown at the bottom chart of Figure 2 show that the hOB-IgG-1 fusion protein substantially reduced food intake. This result was unexpected as it was assumed that the fusion protein would be too large to cross the blood-brain barrier and exert its effect.

Obese *(ob*/*ob)* female mice were treated with either hOB or the hOB-IgG-1 chimera by daily subcutaneous injections for 7 days. The data shown in Figure 3 again demonstrate that the chimera is more effective than the native hOB protein at reducing body weight (top) and food intake (bottom).

In a further experiment, obese *(ob*/*ob)* female mice were treated with either the hOB-IgG-1 fusion protein, native hOB or hCD4-IgG-1 (control) by daily subcutaneous injections for seven days. The results shown in Figure 5 affirm that the hOB-IgG-1 fusion protein is more effective than the native hOB protein at reducing body weight (top and middle panels) and food intake (bottom panel).

### D. Results with PEG-hOB

Obese female *ob*/*ob* mice were treated with human OB protein or with PEG derivatives of human OB. The data are shown in Figure 4. The data presented in the top chart demonstrate that PEG-hOB is more potent than the native hOB protein at reducing body weight, when both proteins are administered similarly by daily subcutaneous infusion.

The data shown at the bottom chart of Figure 4 show that the PEG-hOB proteins were substantially more effective in reducing food intake than unmodified native hOB.

### Reference Example

### Identification and cloning of an OB receptor

The OB protein-immunoadhesinof Example 1 was used to detect and expression clone an OB receptor. First, to identify a receptor source, several cell lines were screened with
1 µg/ml OB-1gG-1 fusion by flow cytometry. The detection system which consists of a biotin conjugated secondary antibody followed by streptavidin-phycoerythrinprovides a dramatic amplification of the signal and allows the detection of cells expressing low numbers of receptors. Two cell lines, human embryonic kidney 293 and human lung A549 cells were found to bind OB-IgG-1 but not an Flt-4 control immunoadhesin. Specific binding of OB-IgG-1 to the cells was also demonstrated by the addition of excess of bacterially expressed human OB protein. Addition of 10 µg/ml of human OB completely blocks the binding of OB-IgG-1 to 293 cells.

To isolate a cDNA encoding the OB receptor, COSN cells were transiently transfected with pools of approximately 10⁵ clones of an oligo dT primed 293 cell cDNA library in pRK5B. Transfected cells were enriched by panning on plates coated with an anti-human Fc antibody after incubation with OB-IgG-1. After three rounds of enrichment, 1 of 30 pools gave rise to OB-IgG-1 mediated adherence of COSN cells to the binding plates which could be competed by human leptin. cDNA clones picked randomly from this third round were transfected in pools of 10-20. Individual clones were finally identified after breaking down one pool of 10 that was scoring positive by panning.

Sequence analysis revealed a clone of approximately 5300 bp with an open reading frame encoding a protein of 896 amino acids. The sequence corresponded to a type 1 transmembrane protein with a 22 amino acid long signal peptide, 819 amino acid extracellulardomain, 21 amino acid transmembrane domain and a short 34 amino acid intracellular domain. The sequence was found to essentially correspond to the human OB receptor identified and isolated by Tartaglia *et al., supra,* and is identical with a human receptor sequence disclosed in copending application Serial No. 08/585,005 filed January 11, 1996.

While the invention has been illustrated by way of examples, the scope of the invention is not so limited. It will be understood that further modifications and variations are possible without diverting from the overall concept of the invention. All such modifications are intended to be within the scope of the present invention.

## Claims (Claims for the following Contracting State(s): BE, CH, LI, DE, DK, ES, FR, GB, IT, NL)

1. A covalent derivative of an OB protein having a longer plasma half-life and/or slower clearance than a corresponding native OB protein and capable of reducing body weight and/or food intake in an individual treated,
which derivative is an OB-immunoglobulin chimera comprising an OB protein amino acid sequence capable of binding to a native OB receptor, linked to an immunoglobulin sequence,
and modified with a hydrophilic nonproteinaceous polymer.

2. The derivative of claim 1 wherein the nonproteinaceous polymer is a polyethylene glycol (PEG).

3. The derivative of claim 1 which is a chimeric polypeptide wherein said immunoglobulin sequence is a constant domain sequence.

4. The derivative of claim 1 which is a chimeric polypeptide wherein said OB protein is human.

5. A composition for the treatment of a condition associated with the abnormal expression or function of the OB gene, or for eliciting a biological response mediated by an OB receptor, comprising an effective amount of an OB derivative of claim 1.

6. The composition of claim 5 effective for weight and/or appetite reduction.

7. The composition of claim 5 effective in the reduction of elevated insulin levels.

8. The composition of claim 5 which comprises an effective amount of the derivative of claim 1 which is a chimeric polypeptide in association with a pharmaceutically acceptable carrier.

9. The derivative of claim 1 for use in a method for the treatment of a condition associated with the abnormal expression or function of the OB gene, or for eliciting a biological response mediated by an OB receptor, comprising administering to an individual to be treated the derivative.

10. The derivative of claim 9 wherein the condition to be treated is selected from the group consisting of obesity, bulemia, and Type I or II diabetes.

11. The derivative of claim 1 for use in a method for inducing weight loss or appetite loss in a subject, comprising administering to said subject an effective amount of the derivative.

12. A chimeric polypeptide comprising a human OB protein amino acid sequence capable of binding to a native OB receptor, linked to an immunoglobulin constant domain sequence.
wherein two OB polypeptide-IgG heavy chain fusions are linked to each other by at least one disulfide bond to yield a homodimeric immunoglobulin-like structure.

13. The chimeric polypeptide of claim 12 wherein at least one of said OB polypeptide-IgG heavy chain fusions is associated with an immunoglobulin light chain.

14. An isolated nucleic acid sequence encoding at least two OB protein-immunoglobulin fusions.

15. A replicable expression vector comprising the nucleic acid of claim 14.

16. A host cell transformed with the replicable expression vector of claim 15.

17. A process comprising culturing the host cells of claim 16 so as to express the nucleic acid encoding the OB protein-immunoglobulin fusions.

18. The process of claim 17 wherein said cells are further transformed with nucleic acid encoding at least one immunoglobulin light chain.

19. A method for increasing the potency of an OB protein which method comprises preparing a derivative of the OB protein by:
(i) fusing said OB protein to an immunoglobulin domain, or
(ii) covalently bonding said OB protein, or an OB protein-immunoglobulin chimera, to a hydrophilic nonproteinaceous polymer.

20. A method as claimed in claim 19 wherein the derivative is more potent at reducing body weight or adipose deposits.

21. A method as claimed in claim 19 wherein the derivative is more potent at reducing food intake.

22. A method as claimed in any one of claims 19 to 21 wherein the polymer is a synthetic polymer.

23. A method as claimed in any one of claims 19 to 22 wherein the OB protein is native OB protein.

24. A method as claimed in claim 23 wherein the native protein is native human OB protein.

25. A method as claimed in claim 22 wherein the nonproteinaceous polymer is a polyethylene glycol (PEG).

26. A method as claimed in any of claims 19 to 25 wherein the OB protein is capable of binding to a native OB receptor and is fused to an immunoglobulin domain.

27. A method as claimed in claim 26 wherein said immunoglobulin sequence is a constant domain sequence.

28. A method as claimed in claim 27 wherein the constant domain is that of an immunoglobulin heavy chain

29. A method as claimed in claim 28 wherein said OB protein is human.

30. A method as claimed in claim 29 wherein two OB polypeptide-IgG heavy chain fusions are linked to each other by at least one disulfide bond to yield a homodimeric immunoglobulin-like structure.

31. A method as claimed in claim 30 wherein at least one of said OB polypeptide-IgG heavy chain fusions is associated with an immunoglobulin light chain.

32. A method as claimed in claim 19 wherein an effective amount of the derivative is admixed with a pharmaceutically acceptable carrier.

33. Use of the derivative of claim 1 for the manufacture of a medicament for the treatment of a condition associated with the abnormal expression or function of the OB gene, or for eliciting a biological response mediated by an OB receptor.

34. The use of claim 33 wherein the condition to be treated is selected from the group consisting of obesity, bulemia, and Type I or II diabetes.

35. The use of claim 33 wherein the treatment is by inducing weight loss or appetite loss in a subject, comprising administering to said subject an effective amount of a derivative of claim 1.

36. The use of claim 33 wherein the treatment is effective in the reduction of elevated insulin levels.

## Claims (Claims for the following Contracting State(s): AT GR, IE, LU, MC, PT, SE, FI)

1. A covalent derivative of an OB protein having a longer plasma half-life and/or slower clearance than a corresponding native OB protein and capable of reducing body weight and/or food intake in an individual treated,
which derivative is an OB-immunoglobulin chimera.

2. A composition for the treatment of a condition associated with the abnormal expression or function of the OB gene, or for eliciting a biological response mediated by an OB receptor, comprising an effective amount of an OB derivative of claim 1.

3. The composition of claim 2 effective for weight and/or appetite reduction.

4. The composition of claim 2 effective in the reduction of elevated insulin levels.

5. A derivative of claim 1 for use in a method for the treatment of a condition associated with the abnormal expression of function of the OB gene, or for eliciting a biological response mediated by an OB receptor, which method comprises administering the derivative to an individual to be treated.

6. The derivative of claim 5 wherein the condition to be treated is selected from the group consisting of obesity, bulemia, and Type I or II diabetes.

7. The derivative of claim 5 wherein the treatment is for inducing weight loss or appetite loss in a subject by administering to said subject an effective amount of a derivative of claim 1.

8. A chimeric polypeptide comprising an OB protein amino acid sequence capable of binding to a native OB receptor, linked to an immunoglobulin sequence.

9. The chimeric polypeptide of claim 8 wherein said immunoglobulin sequence is a constant domain sequence.

10. The chimeric polypeptide of claim 9 wherein said OB protein is human.

11. The chimeric polypeptide of claim 10 wherein two OB polypeptide-IgG heavy chain fusions are linked to each other by at least one disulfide bond to yield a homodimeric immunoglobulin-like structure.

12. The chimeric polypeptide of claim 11 wherein at least one of said OB polypeptide-IgG heavy chain fusions is associated with an immunoglobulin light chain.

13. An isolated nucleic acid sequence encoding an OB protein-immunoglobulin fusion.

14. A replicable expression vector comprising the nucleic acid of claim 13.

15. A host cell transformed with the replicable expression vector of claim 14.

16. A process comprising culturing the host cells of claim 15 so as to express the nucleic acid encoding an OB protein-immunoglobulin fusion.

17. The process of claim 16 wherein said host cells are cotransformed with nucleic acid encoding at least two OB protein-immunoglobulin fusions.

18. The process of claim 17 wherein said cells are further transformed with nucleic acid encoding at least one immunoglobulin light chain.

19. The chimeric polypeptide of claim 8 for use in a method of treating a condition associated with the abnormal expression or function of the OB gene or for eliciting a biological response mediated by an OB receptor, which method comprises administering to the patient a therapeutically effective amount of the chimeric polypeptide.

20. The chimeric polypeptide of claim 19 wherein said condition is selected from the group consisting of obesity, bulemia and type I or II diabetes.

21. A composition for the treatment of obesity comprising an effective amount of a chimeric polypeptide of claim 8 in association with a pharmaceutically acceptable carrier.

22. A method for increasing the potency of an OB protein which method comprises preparing a derivative of the OB protein by:
(i) fusing said OB protein to an immunoglobulin domain, or
(ii) covalently bonding said OB protein, or an OB protein-immunoglobulin chimera, to a hydrophilic nonproteinaceous polymer.

23. A method as claimed in claim 22 wherein the derivative is more potent at reducing body weight or adipose deposits.

24. A method as claimed in claim 22 wherein the derivative is more potent at reducing food intake.

25. A method as claimed in any one of claims 22 to 24 wherein the polymer is a synthetic polymer.

26. A method as claimed in any one of claims 22 to 24 wherein the OB protein is native OB protein.

27. A method as claimed in claim 26 wherein the native protein is native human OB protein.

28. A method as claimed in claim 25 wherein the nonproteinaceous polymer is a polyethylene glycol (PEG).

29. A method as claimed in any of claims 21 to 27 wherein the OB protein is capable of binding to a native OB receptor and is fused to an immunoglobulin domain.

30. A method as claimed in claim 29 wherein said immunoglobulin sequence is a constant domain sequence.

31. A method as claimed in claim 30 wherein the constant domain is that of an immunoglobulin heavy chain

32. A method as claimed in claim 31 wherein said OB protein is human.

33. A method as claimed in claim 32 wherein two OB polypeptide-IgG heavy chain fusions are linked to each other by at least one disulfide bond to yield a homodimeric immunoglobulin-like structure.

34. A method as claimed in claim 33 wherein at least one of said OB polypeptide-IgG heavy chain fusions is associated with an immunoglobulin light chain.

35. A method as claimed in claim 22 wherein an effective amount of the derivative is admixed with a pharmaceutically acceptable carrier.

36. Use of a derivative of claim 1 for the manufacture of a medicament for the treatment of a condition associated with the abnormal expression or function of the OB gene, or for eliciting a biological response mediated by an OB receptor.

37. The use of claim 36 wherein the condition to be treated is selected from the group consisting of obesity, bulemia, and Type or II diabetes.

38. The use of claim 36 wherein the treatment is by inducing weight loss or appetite loss in a subject, comprising administering to said subject an effective amount of a derivative of claim 1.

39. The use of claim 36 wherein the treatment is effective in the reduction of elevated insulin levels.

## Patentansprüche (Patentansprüche für folgende(n) Vertragsstaat(en): BE, CH, LI, DE, DK, ES, FR, GB, IT, NL)

1. Kovalentes Derivat eines OB-Proteins mit einer längeren Plasma halbwertszeit und/oder langsameren Clearance als ein entsprechendes natives OB-Protein, das zur Reduzierung des Körpergewichts und/oder der Nahrungsaufnahme eines behandelten Individuums fähig ist,
wobei das Derivat eine OB-Immunglobulin-Chimäre ist, die eine an eine Immunglobulinsequenz gebundene OB-Protein-Aminosäuresequenz umfasst, die zur Bindung an einen nativen OB-Rezeptor fähig ist,
und mit einem hydrophilen nichtproteinartigen Polymer modifiziert ist.

2. Derivat nach Anspruch 1, worin das nichtproteinartige Polymer ein Polyethylenglykol (PEG) ist.

3. Derivat nach Anspruch 1, das ein chimäres Polypeptid ist, worin die Immunglobulinsequenz eine Konstantdomänensequenz ist.

4. Derivat nach Anspruch 1, das ein chimäres Polypeptid ist, worin das OB-Protein vom Menschen stammt.

5. Zusammensetzung zur Behandlung eines in Zusammenhang mit der abnormalen Expression oder Funktion des OB-Gens stehenden Leidens oder zur Auslösung einer durch einen OB-Rezeptor vermittelten biologischen Reaktion, die eine wirksame Menge eines OB-Derivats nach Anspruch 1 umfasst.

6. Zusammensetzung nach Anspruch 5, die bei Gewichtsreduktion und/oder Appetitzügelung wirksam ist.

7. Zusammensetzung nach Anspruch 5, die bei der Senkung von erhöhten Insulinwerten wirksam ist.

8. Zusammensetzung nach Anspruch 5, die eine wirksame Menge eines Derivats nach Anspruch 1, das ein chimäres Polypeptid ist, zusammen mit einem pharmazeutisch annehmbaren Träger umfasst.

9. Derivat nach Anspruch 1 zur Verwendung in einem Verfahren zur Behandlung eines in Zusammenhang mit der abnormalen Expression oder Funktion des OB-Gens stehenden Leidens oder zur Auslösung einer durch einen OB-Rezeptor vermittelten biologischen Reaktion, das die Verabreichung des Derivats an ein zu behandelndes Idividuum umfasst.

10. Derivat nach Anspruch 9, worin das zu behandelnde Leiden aus der aus Fettleibigkeit, Bulimie und Diabetes vom Typ I oder II bestehenden Gruppe ausgewählt ist.

11. Derivat nach Anspruch 1 zur Verwendung in einem Verfahren zur Herbeiführung von Gewichtsverlust oder Appetitzügelung bei einem Individuum, das die Verabreichung einer wirksamen Menge des Derivats an das Individuum umfasst.

12. Chimäres Polypeptid, umfassend eine menschliche OB-Protein-Aminosäuresequenz, die zur Bindung an einen nativen OB-Rezeptor fähig ist und an eine Immunglobulin-Konstantdomänensequenz gebunden ist,
worin zwei OB-Polypeptid-lgG-Schwerkettenfusionen über zumindest eine Disulfidbindung aneinander gebunden sind, um eine homodimere immunglobulinähnliche Struktur zu erhalten.

13. Chimäres Polypeptid nach Anspruch 12, worin zumindest eine der OB-Polypeptid-IgG-Schwerkettenfusionen mit einer Immunglobulin-Leichtkette verbunden ist.

14. Isolierte Nucleinsäuresequenz, die für zumindest zwei OB-Protein-Immunglobulinfusionen kodiert.

15. Replizierbarer Expressionsvektor, der eine Nucleinsäure nach Anspruch 14 umfasst.

16. Wirtszelle, die mit dem replizierbaren Expressionsvektor nach Anspruch 15 transformiert ist.

17. Verfahren, umfassend das Kultivieren von Wirtszellen nach Anspruch 16, um die Nucleinsäure zu exprimieren, die für die OB-Protein-Immunglobulinfusionen kodiert.

18. Verfahren nach Anspruch 17, worin die Zellen außerdem mit Nucleinsäure transformiert sind, die für zumindest eine Immunglobulin-Leichtkette kodiert.

19. Verfahren zur Erhöhung der Wirksamkeit eines OB-Proteins, wobei das Verfahren die Herstellung eines Derivats des OB-Proteins auf folgende Weise umfasst:
(i) Fusionieren des OB-Proteins an eine Immunglobulindomäne, oder
(ii) kovalente Bindung des OB-Proteins oder einer OB-Protein-Immunglobulin-Chimäre an ein hydrophiles nichtproteinartiges Polymer.

20. Verfahren nach Anspruch 19, worin das Derivat wirksamer bei der Reduzierung von Körpergewicht oder Fettdepots ist.

21. Verfahren nach Anspruch 19, worin das Derivat wirksamer bei der Reduzierung der Nahrungsaufnahme ist.

22. Verfahren nach einem der Ansprüche 19 bis 21, worin das Polymer ein synthetisches Polymer ist.

23. Verfahren nach einem der Ansprüche 19 bis 22, worin das OB-Protein ein natives OB-Protein ist.

24. Verfahren nach Anspruch 23, worin das native Protein ein natives menschliches OB-Protein ist.

25. Verfahren nach Anspruch 22, worin das nichtproteinartige Polymer ein Polyethylenglykol (PEG) ist.

26. Verfahren nach einem der Ansprüche 19 bis 25, worin das OB-Protein zur Bindung an einen nativen OB-Rezeptor fähig ist und an eine lmmunglobulindomäne fusioniert ist.

27. Verfahren nach Anspruch 26, worin die Immunglobulinsequenz eine Konstantdomänensequenz ist.

28. Verfahren nach Anspruch 27, worin die konstante Domäne die einer Immunglobulin-Schwerkette ist.

29. Verfahren nach Anspruch 28, worin das OB-Protein vom Menschen stammt.

30. Verfahren nach Anspruch 29, worin zwei OB-Polypeptid-IgG-Schwerkettenfusionen über zumindest eine Disulfidbindung aneinander gebunden sind, um eine homodimere immunglobulinähnliche Struktur zu erhalten.

31. Verfahren nach Anspruch 30, worin zumindest eine der OB-Polypeptid-IgG-Schwerkettenfusionen mit einer Immunglobulin-Leichtkette verbunden ist.

32. Verfahren nach Anspruch 19, worin eine wirksame Menge des Derivats mit einem pharmazeutisch annehmbaren Träger vermischt wird.

33. Verwendung eines Derivats nach Anspruch 1 zur Herstellung eines Medikaments zur Behandlung eines in Zusammenhang mit der abnormalen Expression oder Funktion des OB-Gens stehenden Leidens oder zur Auslösung einer durch einen OB-Rezeptor vermittelten biologischen Reaktion.

34. Verwendung nach Anspruch 33, worin das zu behandelnde Leiden aus der aus Fettleibigkeit, Bulimie und Diabetes vom Typ I oder II bestehenden Gruppe ausgewählt ist.

35. Verwendung nach Anspruch 33, worin die Behandlung in der Herbeiführung von Gewichtsverlust oder Appetitzügelung bei einem Individuum besteht, umfassend die Verabreichung einer wirksamen Menge eines Derivats nach Anspruch 1 an das Individuum.

36. Verfahren nach Anspruch 33, worin die Behandlung bei der Senkung von erhöhten Insulinwerten wirksam ist.

## Patentansprüche (Patentansprüche für folgende(n) Vertragsstaat(en): AT, GR, IE, LU, MC, PT, SE, FI)

1. Kovalentes Derivat eines OB-Proteins mit einer längeren Plasmahalbwertszeit und/oder langsameren Clearance als ein entsprechendes natives OB-Protein, das zur Reduzierung des Körpergewichts und/oder der Nahrungsaufnahme eines behandelten Individuums fähig ist,
wobei das Derivat eine PB-Immunglobulin-Chimäre ist.

2. Zusammensetzung zur Behandlung eines in Zusammenhang mit der abnormalen Expression oder Funktion des OB-Gens stehenden Leidens oder zur Auslösung einer durch einen OB-Rezeptor vermittelten biologischen Reaktion, die eine wirksame Menge eines OB-Derivats nach Anspruch 1 umfasst.

3. Zusammensetzung nach Anspruch 2, die bei Gewichtsreduktion und/oder Appetitzügelung wirksam ist.

4. Zusammensetzung nach Anspruch 2, die bei der Senkung von erhöhten Insulinwerten wirksam ist.

5. Derivat nach Anspruch 1 zur Verwendung in einem Verfahren zur Behandlung eines in Zusammenhang mit der abnormalen Expression oder Funktion des OB-Gens stehenden Leidens oder zur Auslösung einer durch einen OB-Rezeptor vermittelten biologischen Reaktion, wobei das Verfahren die Verabreichung des Derivats an ein zu behandelndes Individuum umfasst.

6. Derivat nach Anspruch 5, worin das zu behandelnde Leiden aus der aus Fettleibigkeit, Bulimie und Diabetes vom Typ I oder II bestehenden Gruppe ausgewählt ist.

7. Derivat nach Anspruch 5, worin die Behandlung zur Herbeiführung von Gewichtsverlust oder Appetitzügelung bei einem Individuum dient und die Verabreichung einer wirksamen Menge eines Derivats nach Anspruch 1 an das Individuum umfasst.

8. Chimäres Polypeptid, umfassend eine OB-Protein-Aminosäuresequenz, die zur Bindung an einen nativen OB-Rezeptor fähig ist und an eine Immunglobulinsequenz gebunden ist.

9. Chimäres Polypeptid nach Anspruch 8, worin die Immunglobulinsequenz eine Konstantdomänensequenz ist.

10. Chimäres Polypeptid nach Anspruch 9, worin das OB-Protein vom Menschen stammt.

11. Chimäres Polypeptid nach Anspruch 10, worin zwei OB-Polypeptid-IgG-Schwerkettenfusionen über zumindest eine Disulfidbindung aneinander gebunden sind, um eine homodimere immunglobulinähnliche Struktur zu erhalten.

12. Chimäres Polypeptid nach Anspruch 11, worin zumindest eine der OB-Polypeptid-IgG-Schwerkettenfusionen mit einer Immunglobulin-Leichtkette verbunden ist.

13. Isolierte Nucleinsäuresequenz, die für eine OB-Protein-Immunglobulinfusion kodiert.

14. Replizierbarer Expressionsvektor, der eine Nucleinsäure nach Anspruch 13 umfasst.

15. Wirtszelle, die mit einem replizierbaren Expressionsvektor nach Anspruch 14 transformiert ist.

16. Verfahren, umfassend das Kultivieren von Wirtszellen nach Anspruch 15, um die Nucleinsäure zu exprimieren, die für eine OB-Protein-Immunglobulinfusion kodiert.

17. Verfahren nach Anspruch 16, worin die Wirtszellen außerdem mit Nucleinsäure cotransformiert sind, die für zumindest zwei OB-Protein-Immunglobulinfusionen kodiert.

18. Verfahren nach Anspruch 17, worin die Zellen außerdem mit Nucleinsäure transformiert sind, die für zumindest eine Immunglobulin-Leichtkette kodiert.

19. Chimäres Polypeptid nach Anspruch 8 zur Verwendung in einem Verfahren zur Behandlung eines in Zusammenhang mit der abnormalen Expression oder Funktion des OB-Gens stehenden Leidens oder zur Auslösung einer durch einen OB-Rezeptor vermittelten biologischen Reaktion, wobei das Verfahren die Verabreichung einer wirksamen Menge des chimären Polypeptids an einen Patienten umfasst.

20. Chimäres Polypeptid nach Anspruch 19, worin das Leiden aus der aus Fettleibigkeit, Bulimie und Diabetes vom Typ I oder II bestehenden Gruppe ausgewählt ist.

21. Zusammensetzung zur Behandlung von Fettleibigkeit, umfassend eine wirksame Menge eines chimären Polypeptids nach Anspruch 8 zusammen mit einem pharmazeutisch annehmbaren Träger.

22. Verfahren zur Erhöhung der Wirksamkeit eines OB-Proteins, wobei das Verfahren die Herstellung eines Derivats des OB-Proteins auf folgende Weise umfasst:
(i) Fusionieren des OB-Proteins an eine Immunglobulindomäne, oder
(ii) kovalente Bindung des OB-Proteins oder einer OB-Protein-Immunglobulin-Chimäre an ein hydrophiles nichtproteinartiges Polymer.

23. Verfahren nach Anspruch 22, worin das Derivat wirksamer bei der Reduzierung von Körpergewicht oder Fettdepots ist.

24. Verfahren nach Anspruch 22, worin das Derivat wirksamer bei der Reduzierung der Nahrungsaufnahme ist.

25. Verfahren nach einem der Ansprüche 22 bis 24, worin das Polymer ein synthetisches Polymer ist.

26. Verfahren nach einem der Ansprüche 22 bis 24, worin das OB-Protein ein natives OB-Protein ist.

27. Verfahren nach Anspruch 26, worin das native Protein ein natives menschliches OB-Protein ist.

28. Verfahren nach Anspruch 25, worin das nichtproteinartige Polymer ein Polyethylenglykol (PEG) ist.

29. Verfahren nach einem der Ansprüche 21 bis 27, worin das OB-Protein zur Bindung an einen nativen OB-Rezeptor fähig ist und an eine Immunglobulindomänefusioniert ist.

30. Verfahren nach Anspruch 29, worin die Immunglobulinsequenz eine Konstantdomänensequenz ist.

31. Verfahren nach Anspruch 30, worin die konstante Domäne die einer Immunglobulin-Schwerkette ist.

32. Verfahren nach Anspruch 31, worin das OB-Protein vom Menschen stammt.

33. Verfahren nach Anspruch 32, worin zwei OB-Polypeptid-IgG-Schwerkettenfusionen über zumindest eine Disulfidbindung aneinander gebunden sind, um eine homodimere immunglobulinähnliche Struktur zu erhalten.

34. Verfahren nach Anspruch 33, worin zumindest eine der OB-Polypeptid-IgG-Schwerkettenfusionen mit einer Immunglobulin-Leichtkette verbunden ist.

35. Verfahren nach Anspruch 22, worin eine wirksame Menge des Derivats mit einem pharmazeutisch annehmbaren Träger vermischt wird.

36. Verwendung eines Derivats nach Anspruch 1 zur Herstellung eines Medikaments zur Behandlung eines in Zusammenhang mit der abnormalen Expression oder Funktion des OB-Gens stehenden Leidens oder zur Auslösung einer durch einen OB-Rezeptor vermittelten biologischen Reaktion.

37. Verwendung nach Anspruch 36, worin das zu behandelnde Leiden aus der aus Fettleibigkeit, Bulimie und Diabetes vom Typ I oder II bestehenden Gruppe ausgewählt ist.

38. Verwendung nach Anspruch 36, worin die Behandlung in der Herbeiführung von Gewichtsverlust oder Appetitzügelung bei einem Individuum besteht, umfassend die Verabreichung einer wirksamen Menge eines Derivats nach Anspruch 1 an das Individuum.

39. Verfahren nach Anspruch 36, worin die Behandlung bei der Senkung von erhöhten Insulinwerten wirksam ist.

## Revendications (Revendications pour l'(les) Etat(s) contractant(s) suivant(s): BE, CH, LI, DE, DK, ES, FR, GB, IT, NL)

1. Dérivé covalent d'une protéine OB ayant une demi-vie plasmatique plus longue et/ou une clairance plus lente qu'une protéine OB naturelle correspondante et capable de réduire le poids corporel et/ou l'absorption de nourriture chez un individu traité,
dérivé qui est une chimère OB-immunoglobuline comprenant une séquence d'aminoacides de protéine OB capable de se lier à un récepteur de OB naturel, liée à une séquence d'immunoglobuline,
et modifié avec un polymère non protéique hydrophile.

2. Dérivé suivant la revendication 1, dans lequel le polymère non protéique est un polyéthylèneglycol (PEG).

3. Dérivé suivant la revendication 1, qui est un polypeptide chimère dans lequel ladite séquence d'immunoglobuline est une séquence de domaine constant.

4. Dérivé suivant la revendication 1, qui est un polypeptide chimère dans lequel ladite protéine OB est humaine.

5. Composition pour le traitement d'une affection associée à l'expression ou la fonction anormale du gène OB, ou pour engendrer une réponse biologique à médiation par un récepteur de OB, comprenant une quantité efficace d'un dérivé de OB suivant la revendication 1.

6. Composition suivant la revendication 5, efficace pour la réduction du poids et/ou de l'appétit.

7. Composition suivant la revendication 5, efficace dans la réduction de taux élevés d'insuline.

8. Composition suivant la revendication 5, qui comprend une quantité efficace du dérivé suivant la revendication 1 qui est un polypeptide chimère en association avec un support pharmaceutiquement acceptable.

9. Dérivé suivant la revendication 1, destiné à être utilisé dans une méthode pour le traitement d'une affection associée à l'expression ou la fonction anormale du gène OB, ou pour engendrer une réponse biologique à médiation par un récepteur de OB, comprenant l'administration du dérivé à un individu à traiter.

10. Dérivé suivant la revendication 9, dans lequel l'affection à traiter est choisie dans le groupe consistant en l'obésité, la boulimie et le diabète de type I ou II.

11. Dérivé suivant la revendication 1, destiné à être utilisé dans une méthode pour induire une perte de poids ou une perte d'appétit chez un sujet, comprenant l'administration audit sujet d'une quantité efficace du dérivé.

12. Polypeptide chimère comprenant une séquence d'aminoacides de protéine OB humaine capable de se lier à un récepteur de OB naturel, liée à une séquence de domaine constant d'immunoglobuline,
dans lequel deux fusions polypeptide OB-chaîne lourde de IgG sont liées l'une à l'autre par au moins une liaison disulfure pour obtenir une structure analogue à une immunoglobuline homodimère.

13. Polypeptide chimère suivant la revendication 12, dans lequel au moins une desdites fusions polypeptide OB-chaîne lourde de IgG est associée à une chaîne légère d'immunoglobuline.

14. Séquence d'acide nucléique isolée codant pour au moins deux fusions protéine OB-immunoglobuline.

15. Vecteur d'expression réplicable comprenant l'acide nucléique suivant la revendication 14.

16. Cellule hôte transformée avec le vecteur d'expression réplicable suivant la revendication 15.

17. Procédé comprenant la culture des cellules hôtes suivant la revendication 16 de manière à exprimer l'acide nucléique codant pour les fusions protéine OB-immunoglobuline.

18. Procédé suivant la revendication 17, dans lequel lesdites cellules sont en outre transformées avec un acide nucléique codant pour au moins une chaîne légère d'immunoglobuline.

19. Méthode pour augmenter l'activité d'une protéine OB, méthode qui comprend la préparation d'un dérivé de la protéine OB :
par fusion de ladite protéine OB à un domaine d'immunoglobuline, ou
par fusion covalente de ladite protéine OB, ou d'une chimère protéine OB-immunoglobuline, à un polymère non protéique hydrophile.

20. Méthode suivant la revendication 19, dans laquelle le dérivé est plus actif pour réduire le poids corporel ou les dépôts adipeux.

21. Méthode suivant la revendication 19, dans laquelle le dérivé est plus actif pour réduire l'absorption de nourriture.

22. Méthode suivant l'une quelconque des revendications 19 à 21, dans laquelle le polymère est un polymère synthétique.

23. Méthode suivant l'une quelconque des revendications 19 à 22, dans laquelle la protéine OB est la protéine OB naturelle.

24. Méthode suivant la revendication 23, dans laquelle la protéine naturelle est la protéine OB humaine naturelle.

25. Méthode suivant la revendication 22, dans laquelle le polymère non protéique est une polyéthylèneglycol (PEG).

26. Méthode suivant l'une quelconque des revendications 19 à 25, dans laquelle la protéine OB est capable de se lier à un récepteur de OB naturel et est fusionnée à un domaine d'immunoglobuline.

27. Méthode suivant la revendication 26, dans laquelle ladite séquence d'immunoglobuline est une séquence de domaine constant.

28. Méthode suivant la revendication 27, dans laquelle le domaine constant est celui d'une chaîne lourde d'immunoglobuline.

29. Méthode suivant la revendication 28, dans laquelle ladite protéine OB est humaine.

30. Méthode suivant la revendication 29, dans laquelle deux fusions polypeptide OB-chaîne lourde de IgG sont liées l'une à l'autre par au moins une liaison disulfure, ce qui donne une structure analogue à une immunoglobuline homodimère.

31. Méthode suivant la revendication 30, dans laquelle au moins une desdites fusions polypeptide OB-chaîne lourde de IgG est associée à une chaîne légère d'immunoglobuline.

32. Méthode suivant la revendication 19, dans laquelle une quantité efficace du dérivé est mélangée à un support pharmaceutiquement acceptable.

33. Utilisation du dérivé suivant la revendication 1 pour la production d'un médicament destiné au traitement d'une affection associée à l'expression ou la fonction anormale du gène OB, ou pour engendrer une réponse biologique à médiation par un récepteur de OB.

34. Utilisation suivant la revendication 33, dans laquelle l'affection à traiter est choisie dans le groupe consistant en l'obésité, la boulimie et le diabète de type I ou II.

35. Utilisation suivant la revendication 33, dans laquelle le traitement est effectué en induisant une perte de poids ou une perte d'appétit chez un sujet, comprenant l'administration audit sujet d'une quantité efficace d'un dérivé suivant la revendication 1.

36. Utilisation suivant la revendication 33, dans laquelle le traitement est efficace pour réduire des taux élevés d'insuline.

## Revendications (Revendications pour l'(les) Etat(s) contractant(s) suivant(s): AT, GR, IE, LU, MC, PT, SE, FI)

1. Dérivé covalent d'une protéine OB ayant une demi-vie plasmatique plus longue et/ou une clairance plus lente qu'une protéine OB naturelle correspondante et capable de réduire le poids corporel et/ou l'absorption de nourriture chez un individu traité,
dérivé qui est une chimère OB-immunoglobuline.

2. Composition pour le traitement d'une affection associée à l'expression ou la fonction normale du gène OB, ou pour engendrer une réponse biologique à médiation par un récepteur de OB, comprenant une quantité efficace d'un dérivé de OB suivant la revendication 1.

3. Composition suivant la revendication 2, efficace pour la réduction du poids et/ou de l'appétit.

4. Composition suivant la revendication 2, efficace dans la réduction de taux élevés d'insuline.

5. Dérivé suivant la revendication 1, destiné à être utilisé dans une méthode de traitement d'une affection associée à l'expression ou la fonction anormale du gène OB, ou pour engendrer une réponse biologique à médiation par un récepteur de OB, méthode qui comprend l'administration du dérivé à un individu à traiter.

6. Dérivé suivant la revendication 5, dans lequel l'affection à traiter est choisie dans le groupe consistant en l'obésité, la boulimie et le diabète de type I ou II.

7. Dérivé suivant la revendication 5, dans lequel le traitement est destiné à induire une perte de poids ou une perte d'appétit chez un sujet en administrant audit sujet d'une quantité efficace d'un dérivé suivant la revendication 1.

8. Polypeptide chimère comprenant une séquence d'aminoacides de protéine OB capable de se lier à un récepteur de OB naturel, liée à une séquence d'immunoglobuline.

9. Polypeptide chimère suivant la revendication 8, dans lequel ladite séquence d'immunoglobuline est une séquence de domaine constant.

10. Polypeptide chimère suivant la revendication 9, dans lequel ladite protéine OB est humaine.

11. Polypeptide chimère suivant la revendication 10, dans lequel deux fusions polypeptide OB-chaîne lourde de IgG sont liées l'une à l'autre par au moins une liaison disulfure, ce qui donne une structure analogue à une immunoglobuline homodimère.

12. Polypeptide chimère suivant la revendication 11, dans lequel au moins une desdites fusions polypeptide OB-chaîne lourde de IgG est associée à une chaîne légère d'immunoglobuline.

13. Séquence d'acide nucléique isolée codant pour une fusion protéine OB-immunoglobuline.

14. Vecteur d'expression réplicable comprenant l'acide nucléique suivant la revendication 13.

15. Cellule hôte transformée avec le vecteur d'expression réplicable suivant la revendication 14.

16. Procédé comprenant la culture des cellules hôtes suivant la revendication 15 de manière à exprimer l'acide nucléique codant pour une fusion protéine OB-immunoglobuline.

17. Procédé suivant la revendication 16, dans lequel lesdites cellules sont cotransformées avec un acide nucléique codant pour au moins deux fusions protéine OB-immunoglobuline.

18. Procédé suivant la revendication 17, dans lequel lesdites cellules sont en outre transformées avec un acide nucléique codant pour au moins une chaîne légère d'immunoglobuline.

19. Polypeptide chimère suivant la revendication 8, destiné à être utilisé dans une méthode de traitement d'une affection associée à l'expression ou la fonction anormale du gène OB ou pour engendrer une réponse biologique à médiation par un récepteur de OB, méthode qui comprend l'administration au patient d'une quantité thérapeutiquement efficace du polypeptide chimère.

20. Polypeptide chimère suivant la revendication 19, dans lequel ladite affection est choisie dans le groupe consistant en l'obésité, la boulimie et le diabète de type I ou II.

21. Composition pour le traitement de l'obésité, comprenant une quantité efficace d'un polypeptide chimère suivant la revendication 8 en association avec un support pharmaceutiquement acceptable.

22. Méthode pour accroître l'activité d'une protéine OB, méthode qui comprend la préparation d'un dérivé de la protéine OB :
(i) en fusionnant ladite protéine OB à un domaine d'immunoglobuline, ou
(ii) en liant par covalence ladite protéine OB, ou une chimère protéine OB-immunoglobuline à un polymère non protéique hydrophile.

23. Méthode suivant la revendication 22, dans laquelle le dérivé est plus actif pour réduire le poids corporel ou les dépôts adipeux.

24. Méthode suivant la revendication 22, dans laquelle le dérivé est plus actif pour réduire l'absorption de nourriture.

25. Méthode 22 à 24, dans laquelle le polymère est un polymère synthétique.

26. Méthode suivant l'une quelconque des revendications 22 à 24, dans laquelle la protéine OB est une protéine OB naturelle.

27. Méthode suivant la revendication 26, dans laquelle la protéine naturelle est la protéine OB humaine naturelle.

28. Méthode suivant la revendication 25, dans laquelle le polymère non protéique est un polyéthylèneglycol (PEG).

29. Méthode suivant l'une quelconque des revendications 21 à 27, dans laquelle la protéine OB est capable de se lier à un récepteur de OB naturel et est fusionnée à un domaine d'immunoglobuline.

30. Méthode suivant la revendication 29, dans laquelle ladite séquence d'immunoglobuline est une séquence de domaine constant.

31. Méthode suivant la revendication 30, dans laquelle le domaine constant est celui d'une chaîne lourde d'immunoglobuline.

32. Méthode suivant la revendication 31, dans laquelle ladite protéine OB est humaine.

33. Méthode suivant la revendication 32, dans laquelle deux fusions polypeptide OB-chaîne lourde de IgG sont liées l'une à l'autre par au moins une liaison disulfure, ce qui donne une structure analogue à une immunoglobuline homodimère.

34. Méthode suivant la revendication 33, dans laquelle au moins une desdites fusions polypeptides OB-chaîne lourde de IgG est associée à une chaîne légère d'immunoglobuline.

35. Méthode suivant la revendication 22, dans laquelle une quantité efficace du dérivé est mélangée à un support pharmaceutiquement acceptable.

36. Utilisation du dérivé suivant la revendication 1 pour la production d'un médicament destiné au traitement d'une affection associée à l'expression ou la fonction anormale du gène OB, ou pour engendrer une réponse biologique à médiation par un récepteur de OB.

37. Utilisation suivant la revendication 36, dans laquelle l'affection à traiter est choisie dans le groupe consistant en l'obésité, la boulimie et le diabète de type I ou II.

38. Utilisation suivant la revendication 36, dans laquelle le traitement est effectué en induisant une perte de poids ou une perte d'appétit chez un sujet, comprenant l'administration audit sujet d'une quantité efficace d'un dérivé suivant la revendication 1.

39. Utilisation suivant la revendication 36, dans laquelle le traitement est efficace dans la réduction de taux élevés d'insuline.


### SEQUENCE LISTING

(1) GENERAL INFORMATION:
   (i) APPLICANT: Genentech, Inc.
      De Sauvage, Frederic J.
      Levin, Nancy
      Vandlen, Richard L.
   (ii) TITLE OF INVENTION: OB Protein Derivatives
   (iii) NUMBER OF SEQUENCES: 2
   (iv) CORRESPONDENCE ADDRESS:
      (A) ADDRESSEE: Genentech, Inc.
      (B) STREET: 460 Point San Bruno Blvd
      (C) CITY: South San Francisco
      (D) STATE: California
      (E) COUNTRY: USA
      (F) ZIP: 94080
   (v) COMPUTER READABLE FORM:
      (A) MEDIUM TYPE: 3.5 inch, 1.44 Mb floppy disk
      (B) COMPUTER: IBM PC compatible
      (C) OPERATING SYSTEM: PC-DOS/MS-DOS
      (D) SOFTWARE: WinPatin (Genentech)
   (vi) CURRENT APPLICATION DATA:
      (A) APPLICATION NUMBER:
      (B) FILING DATE: 19-Dec-1996
      (C) CLASSIFICATION:
   (vii) PRIOR APPLICATION DATA:
      (A) APPLICATION NUMBER: 08/667184
      (B) FILING DATE: 20-JUN-1996
   (vii) PRIOR APPLICATION DATA:
      (A) APPLICATION NUMBER: 08/579494
      (B) FILING DATE: 27-DEC-1995
   (viii) ATTORNEY/AGENT INFORMATION:
      (A) NAME: Dreger, Ginger R.
      (B) REGISTRATION NUMBER: 33,055
      (C) REFERENCE/DOCKET NUMBER: 985P2PCT
   (ix) TELECOMMUNICATION INFORMATION:
      (A) TELEPHONE: 415/225-3216
      (B) TELEFAX: 415/952-9881
      (C) TELEX: 910/371-7168
(2) INFORMATION FOR SEQ ID NO:1:
   (i) SEQUENCE CHARACTERISTICS:
      (A) LENGTH: 7127 base pairs
      (B) TYPE: Nucleic Acid
      (C) STRANDEDNESS: Double
      (D) TOPOLOGY: Linear
   (xi) SEQUENCE DESCRIPTION: SEQ ID NO:1:
   TTCGAGCTCG CCCGACATTG ATTATTGACT AGTTATTAAT AGTAATCAAT 50
   TACGGGGTCA TTAGTTCATA GCCCATATAT GGAGTTCCGC GTTACATAAC 100
   TTACGGTAAA TGGCCCGCCT GGCTGACCGC CCAACGACCC CCGCCCATTG 150
   ACGTCAATAA TGACGTATGT TCCCATAGTA ACGCCAATAG GGACTTTCCA 200
   TTGACGTCAA TGGGTGGAGT ATTTACGGTA AACTGCCCAC TTGGCAGTAC 250
   ATCAAGTGTA TCATATGCCA AGTACGCCCC CTATTGACGT CAATGACGGT 300
   AAATGGCCCG CCTGGCATTA TGCCCAGTAC ATGACCTTAT GGGACTTTCC 350
   TACTTGGCAG TACATCTACG TATTAGTCAT CGCTATTACC ATGGTGATGC 400
   GGTTTTGGCA GTACATCAAT GGGCGTGGAT AGCGGTTTGA CTCACGGGGA 450
   TTTCCAAGTC TCCACCCCAT TGACGTCAAT GGGAGTTTGT TTTGGCACCA 500
   AAATCAACGG GACTTTCCAA AATGTCGTAA CAACTCCGCC CCATTGACGC 550
   AAATGGGCGG TAGGCGTGTA CGGTGGGAGG TCTATATAAG CAGAGCTCGT 600
   TTAGTGAACC GTCAGATCGC CTGGAGACGC CATCCACGCT GTTTTGACCT 650
   CCATAGAAGA CACCGGGACC GATCCAGCCT CCGCGGCCGG GAACGGTGCA 700
   TTGGAACGCG GATTCCCCGT GCCAAGAGTG ACGTAAGTAC CGCCTATAGA 750
   GTCTATAGGC CCACCCCCTT GGCTTCGTTA GAACGCGGCT ACAATTAATA 800
   CATAACCTTA TGTATCATAC ACATACGATT TAGGTGACAC TATAGAATAA 850
   CATCCACTTT GCCTTTCTCT CCACAGGTGT CCACTCCCAG GTCCAACTGC 900
   ACCTCGGTTC TATCGATATG CATTGGGGAA CCCTGTGCGG ATTCTTGTGG 950
   CTTTGGCCCT ATCTTTTCTA TGTCCAAGCT GTGCCCATCC AAAAAGTCCA 1000
   AGATGACACC AAAACCCTCA TCAAGACAAT TGTCACCAGG ATCAATGACA 1050
   TTTCACACAC GCAGTCAGTC TCCTCCAAAC AGAAAGTCAC CGGTTTGGAC 1100
   TTCATTCCTG GGCTCCACCC CATCCTGACC TTATCCAAGA TGGACCAGAC 1150
   ACTGGCAGTC TACCAACAGA TCCTCACCAG TATGCCTTCC AGAAACGTGA 1200
   TCCAAATATC CAACGACCTG GAGAACCTCC GGGATCTTCT TCACGTGCTG 1250
   GCCTTCTCTA AGAGCTGCCA CTTGCCCTGG GCCAGTGGCC TGGAGACCTT 1300
   GGACAGCCTG GGGGGTGTCC TGGAAGCTTC AGGCTACTCC ACAGAGGTGG 1350
   TGGCCCTGAG CAGGCTGCAG GGGTCTCTGC AGGACATGCT GTGGCAGCTG 1400
   GACCTCAGCC CTGGGTGCGG GGTCACCGAC AAAACTCACA CATGCCCACC 1450
   GTGCCCAGCA CCTGAACTCC TGGGGGGACC GTCAGTCTTC CTCTTCCCCC 1500
   CAAAACCCAA GGACACCCTC ATGATCTCCC GGACCCCTGA GGTCACATGC 1550
   GTGGTGGTGG ACGTGAGCCA CGAAGACCCT GAGGTCAAGT TCAACTGGTA 1600
   CGTGGACGGC GTGGAGGTGC ATAATGCCAA GACAAAGCCG CGGGAGGAGC 1650
   AGTACAACAG CACGTACCGT GTGGTCAGCG TCCTCACCGT CCTGCACCAG 1700
   GACTGGCTGA ATGGCAAGGA GTACAAGTGC AAGGTCTCCA ACAAAGCCCT 1750
   CCCAGCCCCC ATCGAGAAAA CCATCTCCAA AGCCAAAGGG CAGCCCCGAG 1800
   AACCACAGGT GTACACCCTG CCCCCATCCC GGGAAGAGAT GACCAAGAAC 1850
   CAGGTCAGCC TGACCTGCCT GGTCAAAGGC TTCTATCCCA GCGACATCGC 1900
   CGTGGAGTGG GAGAGCAATG GGCAGCCGGA GAACAACTAC AAGACCACGC 1950
   CTCCCGTGCT GGACTCCGAC GGCTCCTTCT TCCTCTACAG CAAGCTCACC 2000
   GTGGACAAGA GCAGGTGGCA GCAGGGGAAC GTCTTCTCAT GCTCCGTGAT 2050
   GCATGAGGCT CTGCACAACC ACTACACGCA GAAGAGCCTC TCCCTGTCTC 2100
   CGGGTAAATG AGTGCGACGG CCCTAGAGTC GACCTGCAGA AGCTTCTAGA 2150
   GTCGACCTGC AGAAGCTTGG CCGCCATGGC CCAACTTGTT TATTGCAGCT 2200
   TATAATGGTT ACAAATAAAG CAATAGCATC ACAAATTTCA CAAATAAAGC 2250
   ATTTTTTTCA CTGCATTCTA GTTGTGGTTT GTCCAAACTC ATCAATGTAT 2300
   CTTATCATGT CTGGATCGAT CGGGAATTAA TTCGGCGCAG CACCATGGCC 2350
   TGAAATAACC TCTGAAAGAG GAACTTGGTT AGGTACCTTC TGAGGCGGAA 2400
   AGAACCAGCT GTGGAATGTG TGTCAGTTAG GGTGTGGAAA GTCCCCAGGC 2450
   TCCCCAGCAG GCAGAAGTAT GCAAAGCATG CATCTCAATT AGTCAGCAAC 2500
   CAGGTGTGGA AAGTCCCCAG GCTCCCCAGC AGGCAGAAGT ATGCAAAGCA 2550
   TGCATCTCAA TTAGTCAGCA ACCATAGTCC CGCCCCTAAC TCCGCCCATC 2600
   CCGCCCCTAA CTCCGCCCAG TTCCGCCCAT TCTCCGCCCC ATGGCTGACT 2650
   AATTTTTTTT ATTTATGCAG AGGCCGAGGC CGCCTCGGCC TCTGAGCTAT 2700
   TCCAGAAGTA GTGAGGAGGC TTTTTTGGAG GCCTAGGCTT TTGCAAAAAG 2750
   CTGTTAATTC GAACACGCAG ATGCAGTCGG GGCGGCGCGG TCCCAGGTCC 2800
   ACTTCGCATA TTAAGGTGAC GCGTGTGGCC TCGAACACCG AGCGACCCTG 2850
   CAGCGACCCG CTTAACAGCG TCAACAGCGT GCCGCAGATC TGATCAAGAG 2900
   ACAGGATGAG GATCGTTTCG CATGATTGAA CAAGATGGAT TGCACGCAGG 2950
   TTCTCCGGCC GCTTGGGTGG AGAGGCTATT CGGCTATGAC TGGGCACAAC 3000
   AGACAATCGG CTGCTCTGAT GCCGCCGTGT TCCGGCTGTC AGCGCAGGGG 3050
   CGCCCGGTTC TTTTTGTCAA GACCGACCTG TCCGGTGCCC TGAATGAACT 3100
   GCAGGACGAG GCAGCGCGGC TATCGTGGCT GGCCACGACG GGCGTTCCTT 3150
   GCGCAGCTGT GCTCGACGTT GTCACTGAAG CGGGAAGGGA CTGGCTGCTA 3200
   TTGGGCGAAG TGCCGGGGCA GGATCTCCTG TCATCTCACC TTGCTCCTGC 3250
   CGAGAAAGTA TCCATCATGG CTGATGCAAT GCGGCGGCTG CATACGCTTG 3300
   ATCCGGCTAC CTGCCCATTC GACCACCAAG CGAAACATCG CATCGAGCGA 3350
   GCACGTACTC GGATGGAAGC CGGTCTTGTC GATCAGGATG ATCTGGACGA 3400
   AGAGCATCAG GGGCTCGCGC CAGCCGAACT GTTCGCCAGG CTCAAGGCGC 3450
   GCATGCCCGA CGGCGAGGAT CTCGTCGTGA CCCATGGCGA TGCCTGCTTG 3500
   CCGAATATCA TGGTGGAAAA TGGCCGCTTT TCTGGATTCA TCGACTGTGG 3550
   CCGGCTGGGT GTGGCGGACC GCTATCAGGA CATAGCGTTG GCTACCCGTG 3600
   ATATTGCTGA AGAGCTTGGC GGCGAATGGG CTGACCGCTT CCTCGTGCTT 3650
   TACGGTATCG CCGCTCCCGA TTCGCAGCGC ATCGCCTTCT ATCGCCTTCT 3700
   TGACGAGTTC TTCTGAGCGG GACTCTGGGG TTCGAAATGA CCGACCAAGC 3750
   GACGCCCAAC CTGCCATCAC GAGATTTCGA TTCCACCGCC GCCTTCTATG 3800
   AAAGGTTGGG CTTCGGAATC GTTTTCCGGG ACGCCGGCTG GATGATCCTC 3850
   CAGCGCGGGG ATCTCATGCT GGAGTTCTTC GCCCACCCCG GGAGATGGGG 3900
   GAGGCTAACT GAAACACGGA AGGAGACAAT ACCGGAAGGA ACCCGCGCTA 3950
   TGACGGCAAT AAAAAGACAG AATAAAACGC ACGGGTGTTG GGTCGTTTGT 4000
   TCATAAACGC GGGGTTCGGT CCCAGGGCTG GCACTCTGTC GATACCCCAC 4050
   CGAGACCCCA TTGGGGCCAA TACGCCCGCG TTTCTTCCTT TTCCCCACCC 4100
   CAACCCCCAA GTTCGGGTGA AGGCCCAGGG CTCGCAGCCA ACGTCGGGGC 4150
   GGCAAGCCCG CCATAGCCAC GGGCCCCGTG GGTTAGGGAC GGGGTCCCCC 4200
   ATGGGGAATG GTTTATGGTT CGTGGGGGTT ATTCTTTTGG GCGTTGCGTG 4250
   GGGTCAGGTC CACGACTGGA CTGAGCAGAC AGACCCATGG TTTTTGGATG 4300
   GCCTGGGCAT GGACCGCATG TACTGGCGCG ACACGAACAC CGGGCGTCTG 4350
   TGGCTGCCAA ACACCCCCGA CCCCCAAAAA CCACCGCGCG GATTTCTGGC 4400
   GCCGCCGGAC GAACTAAACC TGACTACGGC ATCTCTGCCC CTTCTTCGCT 4450
   GGTACGAGGA GCGCTTTTGT TTTGTATTGG TCACCACGGC CGAGTTTCCG 4500
   CGGGACCCCG GCCAGGGCAC CTGTCCTACG AGTTGCATGA TAAAGAAGAC 4550
   AGTCATAAGT GCGGCGACGA TAGTCATGCC CCGCGCCCAC CGGAAGGAGC 4600
   TGACTGGGTT GAAGGCTCTC AAGGGCATCG GTCGAGCGGC CGCATCAAAG 4650
   CAACCATAGT ACGCGCCCTG TAGCGGCGCA TTAAGCGCGG CGGGTGTGGT 4700
   GGTTACGCGC AGCGTGACCG CTACACTTGC CAGCGCCCTA GCGCCCGCTC 4750
   CTTTCGCTTT CTTCCCTTCC TTTCTCGCCA CGTTCGCCGG CTTTCCCCGT 4800
   CAAGCTCTAA ATCGGGGGCT CCCTTTAGGG TTCCGATTTA GTGCTTTACG 4850
   GCACCTCGAC CCCAAAAAAC TTGATTTGGG TGATGGTTCA CGTAGTGGGC 4900
   CATCGCCCTG ATAGACGGTT TTTCGCCCTT TGACGTTGGA GTCCACGTTC 4950
   TTTAATAGTG GACTCTTGTT CCAAACTGGA ACAACACTCA ACCCTATCTC 5000
   GGGCTATTCT TTTGATTTAT AAGGGATTTT GCCGATTTCG GCCTATTGGT 5050
   TAAAAAATGA GCTGATTTAA CAAAAATTTA ACGCGAATTT TAACAAAATA 5100
   TTAACGTTTA CAATTTTATG GTGCAGGCCT CGTGATACGC CTATTTTTAT 5150
   AGGTTAATGT CATGATAATA ATGGTTTCTT AGACGTCAGG TGGCACTTTT 5200
   CGGGGAAATG TGCGCGGAAC CCCTATTTGT TTATTTTTCT AAATACATTC 5250
   AAATATGTAT CCGCTCATGA GACAATAACC CTGATAAATG CTTCAATAAT 5300
   ATTGAAAAAG GAAGAGTATG AGTATTCAAC ATTTCCGTGT CGCCCTTATT 5350
   CCCTTTTTTG CGGCATTTTG CCTTCCTGTT TTTGCTCACC CAGAAACGCT 5400
   GGTGAAAGTA AAAGATGCTG AAGATCAGTT GGGTGCACGA GTGGGTTACA 5450
   TCGAACTGGA TCTCAACAGC GGTAAGATCC TTGAGAGTTT TCGCCCCGAA 5500
   GAACGTTTTC CAATGATGAG CACTTTTAAA GTTCTGCTAT GTGGCGCGGT 5550
   ATTATCCCGT GATGACGCCG GGCAAGAGCA ACTCGGTCGC CGCATACACT 5600
   ATTCTCAGAA TGACTTGGTT GAGTACTCAC CAGTCACAGA AAAGCATCTT 5650
   ACGGATGGCA TGACAGTAAG AGAATTATGC AGTGCTGCCA TAACCATGAG 5700
   TGATAACACT GCGGCCAACT TACTTCTGAC AACGATCGGA GGACCGAAGG 5750
   AGCTAACCGC TTTTTTGCAC AACATGGGGG ATCATGTAAC TCGCCTTGAT 5800
   CGTTGGGAAC CGGAGCTGAA TGAAGCCATA CCAAACGACG AGCGTGACAC 5850
   CACGATGCCA GCAGCAATGG CAACAACGTT GCGCAAACTA TTAACTGGCG 5900
   AACTACTTAC TCTAGCTTCC CGGCAACAAT TAATAGACTG GATGGAGGCG 5950
   GATAAAGTTG CAGGACCACT TCTGCGCTCG GCCCTTCCGG CTGGCTGGTT 6000
   TATTGCTGAT AAATCTGGAG CCGGTGAGCG TGGGTCTCGC GGTATCATTG 6050
   CAGCACTGGG GCCAGATGGT AAGCCCTCCC GTATCGTAGT TATCTACACG 6100
   ACGGGGAGTC AGGCAACTAT GGATGAACGA AATAGACAGA TCGCTGAGAT 6150
   AGGTGCCTCA CTGATTAAGC ATTGGTAACT GTCAGACCAA GTTTACTCAT 6200
   ATATACTTTA GATTGATTTA AAACTTCATT TTTAATTTAA AAGGATCTAG 6250
   GTGAAGATCC TTTTTGATAA TCTCATGACC AAAATCCCTT AACGTGAGTT 6300
   TTCGTTCCAC TGAGCGTCAG ACCCCGTAGA AAAGATCAAA GGATCTTCTT 6350
   GAGATCCTTT TTTTCTGCGC GTAATCTGCT GCTTGCAAAC AAAAAAACCA 6400
   CCGCTACCAG CGGTGGTTTG TTTGCCGGAT CAAGAGCTAC CAACTCTTTT 6450
   TCCGAAGGTA ACTGGCTTCA GCAGAGCGCA GATACCAAAT ACTGTCCTTC 6500
   TAGTGTAGCC GTAGTTAGGC CACCACTTCA AGAACTCTGT AGCACCGCCT 6550
   ACATACCTCG CTCTGCTAAT CCTGTTACCA GTGGCTGCTG CCAGTGGCGA 6600
   TAAGTCGTGT CTTACCGGGT TGGACTCAAG ACGATAGTTA CCGGATAAGG 6650
   CGCAGCGGTC GGGCTGAACG GGGGGTTCGT GCACACAGCC CAGCTTGGAG 6700
   CGAACGACCT ACACCGAACT GAGATACCTA CAGCGTGAGC ATTGAGAAAG 6750
   CGCCACGCTT CCCGAAGGGA GAAAGGCGGA CAGGTATCCG GTAAGCGGCA 6800
   GGGTCGGAAC AGGAGAGCGC ACGAGGGAGC TTCCAGGGGG AAACGCCTGG 6850
   TATCTTTATA GTCCTGTCGG GTTTCGCCAC CTCTGACTTG AGCGTCGATT 6900
   TTTGTGATGC TCGTCAGGGG GGCGGAGCCT ATGGAAAAAC GCCAGCTGGC 6950
   ACGACAGGTT TCCCGACTGG AAAGCGGGCA GTGAGCGCAA CGCAATTAAT 7000
   GTGAGTTACC TCACTCATTA GGCACCCCAG GCTTTACACT TTATGCTTCC 7050
   GGCTCGTATG TTGTGTGGAA TTGTGAGCGG ATAACAATTT CACACAGGAA 7100
   ACAGCTATGA CCATGATTAC GAATTAA 7127
(2) INFORMATION FOR SEQ ID NO:2:
   (i) SEQUENCE CHARACTERISTICS:
      (A) LENGTH: 397 amino acids
      (B) TYPE: Amino Acid
      (D) TOPOLOGY: Linear
   (xi) SEQUENCE DESCRIPTION: SEQ ID NO:2:
   Met His Trp Gly Thr Leu Cys Gly Phe Leu Trp Leu Trp Pro Tyr
   1 5 10 15
   Leu Phe Tyr Val Gln Ala Val Pro Ile Gln Lys Val Gln Asp Asp
   20 25 30
   Thr Lys Thr Leu Ile Lys Thr Ile Val Thr Arg Ile Asn Asp Ile
   35 40 45
   Ser His Thr Gln Ser Val Ser Ser Lys Gln Lys Val Thr Gly Leu
   50 55 60
   Asp Phe Ile Pro Gly Leu His Pro Ile Leu Thr Leu Ser Lys Met
   65 70 75
   Asp Gln Thr Leu Ala Val Tyr Gln Gln Ile Leu Thr Ser Met Pro
   80 85 90
   Ser Arg Asn Val Ile Gln Ile Ser Asn Asp Leu Glu Asn Leu Arg
   95 100 105
   Asp Leu Leu His Val Leu Ala Phe Ser Lys Ser Cys His Leu Pro
   110 115 120
   Trp Ala Ser Gly Leu Glu Thr Leu Asp Ser Leu Gly Gly Val Leu
   125 130 135
   Glu Ala Ser Gly Tyr Ser Thr Glu Val Val Ala Leu Ser Arg Leu
   140 145 150
   Gln Gly Ser Leu Gln Asp Met Leu Trp Gln Leu Asp Leu Ser Pro
   155 160 165
   Gly Cys Gly Val Thr Asp Lys Thr His Thr Cys Pro Pro Cys Pro
   170 175 180
   Ala Pro Glu Leu Leu Gly Gly Pro Ser Val Phe Leu Phe Pro Pro
                       185                 190                 195
   Lys Pro Lys Asp Thr Leu Met Ile Ser Arg Thr Pro Glu Val Thr
                       200                 205                 210
   Cys Val Val Val Asp Val Ser His Glu Asp Pro Glu Val Lys Phe
                       215                 220                 225
   Asn Trp Tyr Val Asp Gly Val Glu Val His Asn Ala Lys Thr Lys
                       230                 235                 240
   Pro Arg Glu Glu Gln Tyr Asn Ser Thr Tyr Arg Val Val Ser Val
                       245                 250                 255
   Leu Thr Val Leu His Gln Asp Trp Leu Asn Gly Lys Glu Tyr Lys
                       260                 265                 270
   Cys Lys Val Ser Asn Lys Ala Leu Pro Ala Pro Ile Glu Lys Thr
                       275                 280                 285
   Ile Ser Lys Ala Lys Gly Gln Pro Arg Glu Pro Gln Val Tyr Thr
                       290                 295                 300
   Leu Pro Pro Ser Arg Glu Glu Met Thr Lys Asn Gln Val Ser Leu
                       305                 310                 315
   Thr Cys Leu Val Lys Gly Phe Tyr Pro Ser Asp Ile Ala Val Glu
                       320                 325                 330
   Trp Glu Ser Asn Gly Gln Pro Glu Asn Asn Tyr Lys Thr Thr Pro
                       335                 340                 345
   Pro Val Leu Asp Ser Asp Gly Ser Phe Phe Leu Tyr Ser Lys Leu
                       350                 355                 360
   Thr Val Asp Lys Ser Arg Trp Gln Gln Gly Asn Val Phe Ser Cys
                       365                 370                 375
   Ser Val Met His Glu Ala Leu His Asn His Tyr Thr Gln Lys Ser
                       380                 385                 390
   Leu Ser Leu Ser Pro Gly Lys
                       395     397